(19) **Europäisches Patentamt**
**European Patent Office**
**Office européen des brevets**

(11) **EP 1 675 519 B1**

(12) **EUROPEAN PATENT SPECIFICATION**

(45) Date of publication and mention
of the grant of the patent:
**23.01.2013 Bulletin 2013/04**

(21) Application number: **04787065.4**

(22) Date of filing: **30.09.2004**

(51) Int Cl.:
***A61B 18/18*** (2006.01)

(86) International application number:
**PCT/EP2004/010928**

(87) International publication number:
**WO 2005/034783 (21.04.2005 Gazette 2005/16)**

(54) **DEVICE AND METHOD FOR THE TREATMENT OF HOLLOW ANATOMICAL STRUCTURES**

VORRICHTUNG UND VERFAHREN ZUR BEHANDLUNG VON HOHLEN ANATOMISCHEN
STRUKTUREN

DISPOSITIF ET METHODE DE TRAITEMENT DE STRUCTURES ANATOMIQUES CREUSES

(84) Designated Contracting States:
**AT BE BG CH CY CZ DE DK EE ES FI FR GB GR
HU IE IT LI LU MC NL PL PT RO SE SI SK TR**

(30) Priority: **03.10.2003 GB 0323158
13.07.2004 GB 0415638**

(43) Date of publication of application:
**05.07.2006 Bulletin 2006/27**

(73) Proprietor: **UK Investment Associates LLC
Carson City, NV 89706 (US)**

(72) Inventors:
• **CRONIN, Nigel
Bath BA2 2ED (GB)**

• **GUY, Adam
Cambridge CB4 0DW (GB)**

(74) Representative: **Evans, Huw David Duncan et al
Chapman Molony
Cardiff Business Technology Centre
Senghennydd Road
Cardiff CF24 4AY (GB)**

(56) References cited:
**WO-A-00/49957      US-A- 5 693 082
US-A- 5 800 494      US-A- 5 810 803
US-A- 6 026 331      US-A1- 2003 187 369**

**Description**

The present invention relates to techniques involved in the thermal ablative therapeutic treatment of the human body, and more particularly to treatment of hollow anatomical structures, for example varicose veins.

Most proposed treatments for varicose veins can be divided into the categories of schlerosing, mechanical manipulation, incision and removal of vein sections, and ligation. There are numerous examples of these in the art, and there are drawbacks associated with each.

Published European patent application EP-A-1,103, 228 discloses a technique for treating vein defects in which a probe connected to a source of high frequency energy is introduced into a vein.

Thermal ablative therapies may be defined as techniques that intentionally decrease body tissue temperature (hypothermia) or intentionally increase body tissue temperature (hyperthermia) to temperatures required for cytotoxic effect, or other therapeutic temperatures required for a particular treatment.

The invention is concerned with hyperthermic thermal ablative therapies. Examples of these include RF, Laser, Focussed (or Ultra-High Speed) Ultrasound, and microwave treatments.

Microwave thermal ablation relies on the fact that microwaves form part of the electromagnetic spectrum causing heating due to interaction between water molecules and the microwave radiation, the heat being used as the cytotoxic mechanism. Treatment involves the introduction of an applicator into the tumours. Microwaves are released from the applicator forming a field around its tip. Direct heating of the water molecules in particular occurs in the radiated microwave field produced around the applicator rather than by conduction from the probe itself. Heating is therefore not reliant on conduction through tissues and cytotoxic temperature levels are reached rapidly.

WO99/56642 discloses a microwave applicator for applying electromagnetic radiation at microwave frequency comprising a coaxial input for a microwave signal input, a waveguide for receiving and propagating the microwave signal input, dielectric material positioned within the waveguide and extending beyond the waveguide to form an antenna for radiating microwave energy, wherein the coaxial input has direct in-line transition to the dielectric-filled waveguide. This direct in-line transition may be achieved by the central conductor of the coaxial input extending axially centrally into the waveguide so as to excite microwaves in the waveguide. A lateral conductor extends radially from the central conductor to assist the launch of the microwaves into the waveguide. The applicator may include a temperature sensor that is directly connected to the coaxial input. Another design of radiation applicator is disclosed in WO00/49957.

W09956643 discloses a method of positioning on a microwave waveguide a sensor including an elongate metallic element comprising: selecting a tubular waveguide; determining the general orientation of the magnetic field generated during microwave transmission; and positioning the elongate metallic element substantially parallel to the orientation of the magnetic field.

Connections of the sensor extend longitudinally of the waveguide and are connected to the outer wall of the waveguide and the central conductor of the coaxial cable that powers the waveguide.

US 5,800,494 discloses an applicator for applying radiation to hollow anatomical structures, the applicator comprising an elongate member which includes an emitter coupled to a source of microwave radiation, the emitter including a radiation emitting portion made of dielectric material and being shaped and dimensioned so as to emit said radiation at a predetermined intensity in a field of limited dimensions adjacent thereto, and an elongate conductor for connecting the radiation emitting portion to the source of microwave radiation.

US 5,810,803 discloses an applicator for applying radiation to hollow anatomical structures, the applicator comprising an elongate member which includes an emitter coupled to a source of microwave radiation, the emitter including a radiation emitting portion made of dielectric material and being shaped and dimensioned so as to emit said radiation at a predetermined intensity in a field of limited dimensions adjacent thereto, and an elongate conductor for connecting a coiled antenna of the radiation emitting portion to the source of microwave radiation.

US 5,693,082 discloses an applicator for applying radiation to hollow anatomical structures, the applicator comprising an elongate member which includes an emitter coupled to a source of microwave radiation, the emitter including a radiation emitting portion made of dielectric material and being shaped and dimensioned so as to emit said radiation at a predetermined intensity in a field of limited dimensions adjacent thereto, and an elongate conductor within and extending at least partially along the radiation emitting portion.

There remains a need for techniques for varicose vein treatment that are effective, minimally invasive, avoid unnecessary surgery, and that are safe and easily controllable by the medical professional.

In accordance with the present invention there is provided a system for the treatment of hollow anatomical structures, the system comprising an applicator for applying radiation to hollow anatomical structures, the applicator comprising:

an elongate member which includes an emitter, the emitter being coupled to a source of microwave radiation and being adapted to emit said radiation, and the emitter including:

a radiation emitting portion made of dielectric material and having an axis of elongation, the radiation emitting portion being shaped and dimensioned so as to emit said radiation at a predetermined intensity in a field of limited dimensions adjacent thereto, whereby occlusion of the tissue of a hollow anatomical structure within said field is effectively accomplished, and

an elongate conductor within and extending at least partially along the radiation emitting portion.

characterised in that_the system further comprises:

a motion rate sensor arranged, in use, for detecting the rate of movement of the applicator; and,

a control unit coupled to the sensor for receiving the motion rate signals output thereby,

wherein the control unit is configured to calculate the speed of motion of the applicator using said motion rate signals, and control the amount of radiation supplied to the applicator and/or the rate of motion of the applicator in dependence upon said calculated speed of motion.

Preferably, the radiation emitting portion includes a generally conical tapering portion, the tapering portion thereby forming a tip for insertion into a hollow anatomical structure.

In one embodiment, the elongate conductor extends along the entire length of the radiation emitting portion, whereby said field is disposed, in use, substantially around said tip.

In another embodiment, the elongate conductor extends partially along the length of the radiation emitting portion, whereby said field is disposed, in use, substantially around the midsection of said radiation emitting portion and spaced apart from said tip.

Preferably, a temperature sensor is provided on said elongate member, said temperature sensor preferably comprising a thermocouple or a fibre optic sensor.

Preferably, radiation emitting portion includes a substantially cylindrical portion integral with the tapering portion.

Preferably, for calculating the speed of motion of the applicator, the control unit is configured to poll the sensor, the polling interval between successive polls being of uniform duration; determine a difference value, the difference value being a difference between counts defined by successive motion rate signals; using the determined difference value and a conversion factor R, calculate the speed of motion of the applicator using R and the difference value.

Preferably, the speed of motion is calculated using $v = (c_j - c_j,) R$ where $(c_i - c_{i-1})$ is the difference value.

Preferably, the applicator is mounted on the end of an elongate cable, and the speed of motion of the applicator is calculated by calculating the speed of motion of the cable. Preferably, the polling interval between successive polls is T, and the conversion factor is determined as $R = 1/KT$, where K is a predetermined count conversion constant for the cable.

Preferably, the cable is a coaxial cable, and a portion of said cable in abutment with the radiation emitting portion is surrounded by, and attached thereto, by a conductive ferrule; and the temperature sensor is disposed on the ferrule.

In one embodiment, a series of regularly spaced markings are provided on the exterior surface of the coaxial cable along its length.

In another embodiment, the markings are non-regularly spaced instead of regularly spaced.

Preferably, the markings comprise alternately light and dark coloured sections.

Preferably, the light and dark coloured sections are each about 1 cm long.

Preferably, the system further includes a display device adapted to display, under the control of the control unit, the calculated speed of motion of the applicator. Preferably, the display device is adapted to display, under the control of the control unit, a graphical representation of the calculated speed of motion of the applicator. Preferably, said graphical representation comprises a speedometer-like graphical representation.

Preferably, the motion rate sensor comprises a housing relative to which, in use, the cable moves, a detection unit disposed within the housing, the detection unit including a conversion device adapted for generating detector signals caused by the motion of the article, and processing circuitry adapted for receiving said detector signals and outputting motion signals indicative of the rate of movement of the article. Preferably, the housing includes at least one aperture permitting motion of the cable relative to the housing. Preferably, the housing has a configuration whereby, in use, the movement of the cable in or near the housing is substantially linear.

Preferably, said at least one aperture includes an entry aperture through which, in use, the cable enters the housing, and an exit aperture through which, in use, the cable exits the housing, the cable preferably moving, in use, in a substantially linear path between said entry aperture and exit aperture.

In one embodiment, the conversion device comprises of at least one radiation detector adapted for receiving radiation from the cable and generating detector signals in dependence on said received radiation. Preferably, the radiation is optical radiation, the detection unit further includes an optical emitter for emitting the optical radiation, and the radiation detector is disposed so as to receive said optical radiation after reflection from the cable. Preferably, the optical emitter is a LED, and preferably wherein the optical emitter and radiation detector comprise an integral device.

In one embodiment, the radiation detector comprises a detector of low-level radioactivity, and the cable has a plurality of radioactive elements disposed therein or thereon in a repetitive pattern along its length.

In another embodiment, the conversion unit includes a magnetic detector, and the cable has a plurality of magnetic elements disposed therein or thereon in a repetitive pattern along its length, the magnetic detector being adapted to generate said detector signals when the cable, in use, moves past the magnetic detector.

In another embodiment, the conversion unit includes one or more rotatable members, such as one or more wheels or balls, adapted to contact the cable and be rotated thereby, in use, and an electromechanical device adapted to generate said detector signals in dependence upon the rate of rotation of said rotatable member (s).

An advantage of the invention is that the dielectric tip (radiation emitting portion) of the elongate member or probe is designed to emit microwaves; and when the microwave power is applied the probe is withdrawn at a certain rate which, in conjunction with the chosen power output, gives a certain, essentially predictable, depth of thermal penetration into the surrounding tissue.

According to another aspect of the invention there is provided a system for the treatment of hollow anatomical structures, comprising: an applicator according to any of claims 39 to 51 of the appended claims; and a motion rate sensor arranged, in use, for detecting the rate of movement of the applicator; a control unit coupled to the sensor for receiving the motion rate signals output thereby; wherein the control unit is configured for calculating the speed of motion of the applicator using said motion rate signals, and control the amount of radiation supplied to the applicator and/or the rate of motion of the applicator in dependence upon said calculated speed of motion.

Preferably, for calculating the speed of motion of the applicator, the control unit is configured to poll the sensor, the polling interval between successive polls being of uniform duration; determine a difference value, the difference value being a difference between counts defined by successive motion rate signals; using the determined difference value and a conversion factor R, calculate the speed of motion of the applicator using R and the difference value.

Preferably, the speed of motion is calculated using

$$v = (c_i - c_{i-1})R$$

where $(c_i - c_{i-1})$ is the difference value.

Preferably, the applicator is mounted on the end of an elongate cable, and the speed of motion of the applicator is calculated by calculating the speed of motion of the cable. Preferably, the polling interval between successive polls is T, and the conversion factor is determined as $R = 1/KT$, where K is a predetermined count conversion constant for the cable.

Preferably, the system further includes a display device adapted to display, under the control of the control unit, the calculated speed of motion of the applicator. Preferably, the display device is adapted to display, under the control of the control unit, a graphical representation of the calculated speed of motion of the applicator. Preferably, said graphical representation comprises a speedometer-like graphical representation.

Preferably, the motion rate sensor comprises a housing relative to which, in use, the cable moves, a detection unit disposed within the housing, the detection unit including a conversion device adapted for generating detector signals caused by the motion of the article, and processing circuitry adapted for receiving said detector signals and outputting motion signals indicative of the rate of movement of the article. Preferably, the housing includes at least one aperture permitting motion of the cable relative to the housing. Preferably, the housing has a configuration whereby, in use, the movement of the cable in or near the housing is substantially linear.

Preferably, said at least one aperture includes an entry aperture through which, in use, the cable enters the housing, and an exit aperture through which, in use, the cable exits the housing, the cable preferably moving, in use, in a substantially linear path between said entry aperture and exit aperture.

In one embodiment, the conversion device comprises of at least one radiation detector adapted for receiving radiation from the cable and generating detector signals in dependence on said received radiation. Preferably, the radiation is optical radiation, the detection unit further includes an optical emitter for emitting the optical radiation, and the radiation detector is disposed so as to receive said optical radiation after reflection from the cable. Preferably, the optical emitter is a LED, and preferably wherein the optical emitter and radiation detector comprise an integral device.

Preferably, the cable has a plurality of markings or reflective elements disposed on the surface thereof in a repetitive pattern along its length.

In one embodiment, the radiation detector comprises a detector of low-level radioactivity, and the cable has a plurality of radioactive elements disposed therein or thereon in a repetitive pattern along its length.

In another embodiment, the conversion unit includes a magnetic detector, and the cable has a plurality of magnetic elements disposed therein or thereon in a repetitive pattern along its length, the magnetic detector being adapted to generate said detector signals when the cable, in use, moves past the magnetic detector.

In another embodiment, the conversion unit includes one or more rotatable members, such as one or more wheels or balls, adapted to contact the cable and be rotated thereby, in use, and an electromechanical device adapted to generate said detector signals in dependence upon the rate of rotation of said rotatable member(s).

An advantage of the invention is that the dielectric tip (radiation emitting portion) of the elongate member or probe is designed to emit microwaves; and when the microwave power is applied the probe is withdrawn at a certain rate which, in conjunction with the chosen power output, gives a certain, essentially predictable, depth of thermal penetration into the surrounding tissue.

A further advantage is that, because the microwaves heat the surrounding tissue directly, there is no need to wait for the heat to penetrate the vein wall.

An additional advantage is that a wide range of treatment rates is possible with the correct combination of power, withdrawal rate and choice of frequency. In addition, treatments of vein defects may have an intensity profile whereby the radiation intensity is suitably varied along the length of the defect.

Embodiments of the invention will now be described, by way of example, with reference to the accompanying drawings, in which:

Figure 1 (PRIOR ART) is a general schematic diagram of the radiation delivery system that may be used in accordance with one aspect of the present invention;

Figure 2 shows (a) a cross-sectional view, (b) an exploded view, and (c) a plot of the resulting radiation field pattern, for a first embodiment of a radiation applicator or probe that may be employed according to one aspect of the invention;

Figure 3 shows a cross-sectional view of a second embodiment of the probe;

Figure 4 shows (a) a cross-sectional view, (b) an exploded view, and (c) a plot of the resulting radiation field pattern, for of a third embodiment of the probe;

Figure 5 shows a cross-sectional view of a fourth embodiment of the probe;

Figure 6 shows (a) a cross-sectional view, and (b) a plot of the resulting radiation field pattern, for a fifth embodiment of the probe;

Figure 7 illustrates (a) an overall view, (b) a close-up view, and (c) a schematic view in use, for an embodiment of the cable employed in the implementation of one aspect of the present invention;

Figures 8(a) to 8(d) depict schematically the movement of the probe in the treatment, according to one aspect of the invention, of a varicose vein;

Figure 9 illustrates one embodiment of the probe, indicating the position of the temperature sensor;

Figure 10 illustrates by flow chart the operation of the software employed on the user's control computer in implementing the invention;

Figure 11 shows arrangement(s) for handling the withdrawal of the cable and probe in alternative embodiments of the invention;

Figure 12 is (a) a close-up perspective view, and (b) an exploded perspective view of the motion rate sensor of Fig. 1;

Figure 13 schematically illustrates a system, in accordance with one aspect of the invention, for performing the controlled movement of the probe and cable, using the sensor of Figs 11 and 12;

Figure 14 illustrates schematically in more detail the communication between the motion rate sensor and the control module in the system of Fig. 13;

Figure 15 illustrates an example of a user interface view displayed to the user by the system of Fig. 13; and

Figure 16 shows an alternative embodiment of the motion rate sensor - in the case where two cables are.used.

It will be appreciated by persons skilled in the art that the electronic systems employed, in accordance with the present invention, to generate, deliver and control the application of radiation to parts of the human body, and the applicator construction, may be as described in the art heretofore. In particular such systems as are described in commonly owned published international patent applications WO95/04385, WO99/56642 and WO00/49957 may be employed (except with the modifications described hereinafter): full details of these systems have been omitted from the following for the sake of brevity.

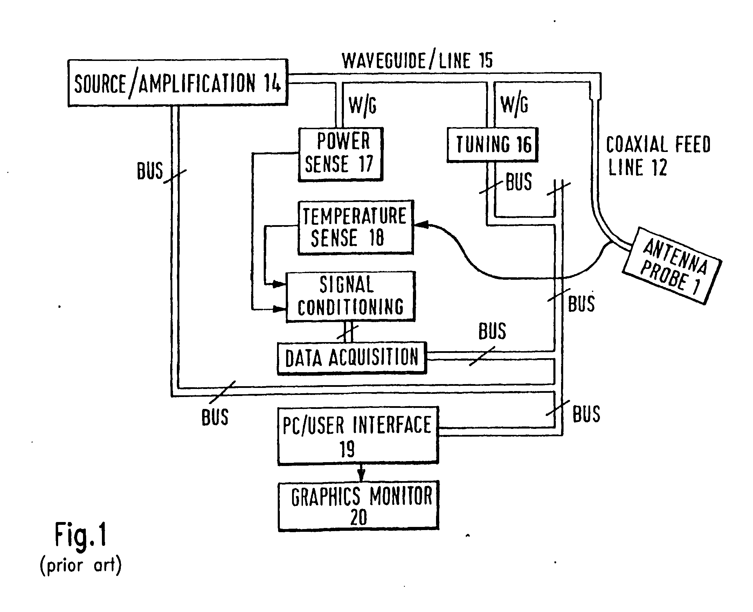

Turning to Fig. 1, this is a general schematic diagram of the radiation delivery system that may be used to implement the present invention. The probe 1 is supplied with a microwave frequency input in the microwave spectrum, preferably in the region of 1-12 GHz, from a microwave frequency generator source and amplifier 14. The amplified signal is passed to the probe 1 via the waveguide line 15 and the coaxial feed line 12. Although the provision of stubs (not shown) may permit tuning of the probe to the specific load, fine tuning is provided by the tuning network 16: this controls the fine tuning of the match of power into the loaded probe. A tuning network may advantageously be used in the invention to ensure that the minimum amount of power is reflected throughout the treatment. An arbitrary level of, for example, less than 10% reflected power from the radiating portion of the elongate member is taken as acceptable.

However, in preferred embodiments of the invention, careful choice of the dimensions and properties of the dielectric, and of the length of the central conductor, of the probe 1 means that probe 1 is optimised to match the tissue to be treated, obviating the need for tuning network 16. The power level of the source/amplification unit 14 is monitored by a power sensor on the waveguide line 15. A thermometry unit is provided to take the temperature sensor readings at the probe/tissue interface. The various signals are collated and conditioned and fed into a PC/user interface 19 which may interface with a user's conventional PC graphics monitor 20. In this way, the user may vary the frequency of the source 14, set the power level required, and vary the tuning network 16 to achieve optimum match into a load. Also during treatment, real-time graphs of temperature data can be viewed on the monitor 20.

The methodology of treating hollow anatomical structures, such as varicose veins, will be discussed in detail later in this disclosure. First, the configuration of various radiation applicators or probes (hereafter "probe") that may be employed in such treatments will be described.

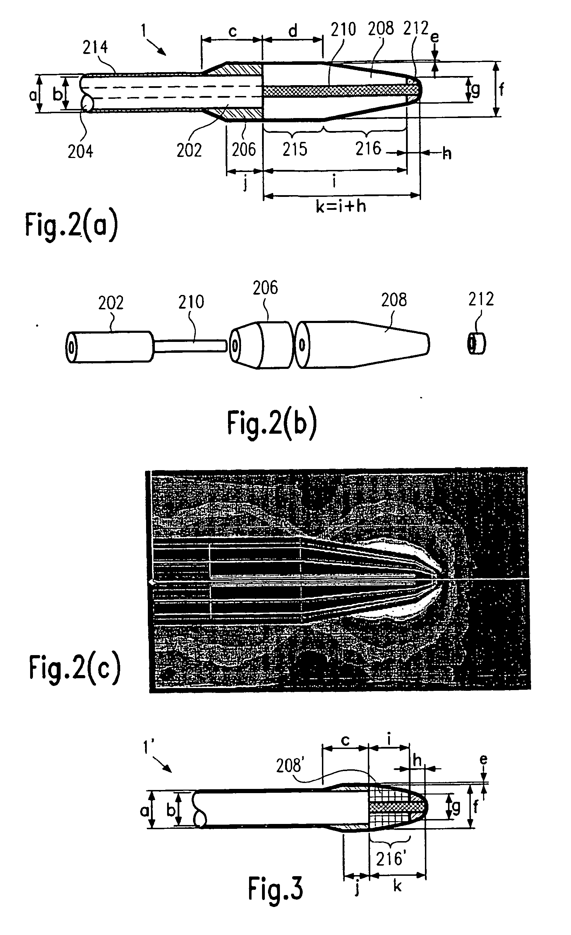

Figure 2 shows views of a first embodiment of a probe that may be employed according to one aspect of the invention. In Fig. 2(a) there is shown the probe generally designated 1 that comprises the end portion 202 of the coaxial cable 204 supplying microwave radiation from the previously described source, a ferrule 206 around the end portion 202, a dielectric member 208, a conductor 210 within the dielectric member 208 and formed by a protruding section of the internal conductor of the coaxial cable 204, and, in this embodiment, a tip member 212. Optionally, the probe 1 may include an outer protective sheath 214, for example made of fluorinated ethylene propylene (FEP). In use, the microwave radiation supplied via cable 204 is emitted by the dielectric member 208 into adjacent tissue: the dielectric member thus forms a monopolar radiating tip designed to radiate at the chosen frequency.

Probes of 3.4mm and 4.8mm diameter have been constructed; however, other diameters are possible depending on the size of the hollow anatomical structures, but will typically be less than 1.2cm. The diameters and lengths of the dielectric member 208 depend on the chosen dielectric properties, the length of the central conductor 210, the frequency of operation and the required diameter of the probe 1. The exact dimensions are chosen so as to minimise reflection when the probe is in tissue.

The power radiated at the tip of the probe 1 by the dielectric member is typically about 1.3 Watts per mm of circumference. With regard to the dimensions mentioned in the aforementioned drawings, the circumference is equal to $\pi(f+2e)$. Larger applicators may need slightly more power to radiate the same depth of thermal penetration; for example, a 3.4 mm dia. probe can radiate 15.1 W total output (1.4 W/mm of circumference), and a 4.8 mm dia. probe can radiate 20 W total output (1.3 W/mm of circumference). Larger applicators radiate more total power, but slightly less power per mm of circumference to achieve the same depth of thermal penetration.

A temperature sensor (not shown), in the form of a thermocouple linked to the aforementioned control system, is preferably provided on the probe: this is suitably disposed on the ferrule 206 and insulated therefrom, for example by plastic tape. (Alternatively, the temperature sensor may be a fibre optic device. In this case, since the fibre optic is not directly heated by the microwave field, it could also be placed on the dielectric member.)

In this embodiment, the dielectric member 208 includes a dielectric generally cylindrical portion 215 and a dielectric generally conical tapering portion 216.

The dimensions may be defined as follows.

    a = thickness of coaxial cable 204 (including sheath)
    b = thickness of coaxial cable 204 (without sheath)
    c = overall length of the ferrule 206
    d = length of non-tapered, cylindrical portion 215 of dielectric member 208
    e = thickness of sheath 214
    f = diameter of ferrule 206 and dielectric member 208 (not including sheath 214)
    g = diameter of metal tip member 212
    h = length of metal tip member 212
    i = total length of dielectric member 208
    j = length of non-tapered part of ferrule 206
    k = length of central conductor 210 that extends beyond the insulated part of the coaxial cable 204.

Figure 2(b) is an exploded perspective view of the probe 1, showing its main constituent parts.

The outer conductor (not shown) of the coaxial cable 204 may be electrically connected to the ferrule 206, which is made of a conductive material such as aluminium. The cable 204 may feed microwave radiation to the probe 1 at various frequencies, and preferably 9.2 GHz in this embodiment. The dielectric member 208 may be made of a known dielectric material, such as Hik 500f, available from Emerson & Cummings, or a hard ceramic dielectric material, such as zirconia (TECHNOX®) which has a permittivity K=25. However, in this embodiment the material is a dielectric material (polyaryletheretherketone (PEEK™)) with permittivity K=3.4. The tip member 212 is made of copper and fixedly attached with, and in good electrical contact with, the end of the conductor 210, for example by soldering or by gluing with an electrically conductive adhesive.

The dimensions (in mm) of the probe 1 in this embodiment are as follows.

| a | b | c | d | e | f | g | h | i | j | k |
|---|---|---|---|---|---|---|---|---|---|---|
| 3.1 | 2.7 | 5 | 5 | 0.2 | 4.4 | 4.4 | 1.1 | 11.6 | 3 | 12.7 |

Figure 2(c) is a view of the radiation field pattern generated by the probe of Fig. 2(a) in use. Lighter areas indicate greater intensity: it can be seen that in this embodiment the maximum intensity (white area) is located near the very tip of the probe 1. This embodiment is intended to achieve instant occlusion of the hollow anatomical structure.

Figure 3 shows a second embodiment of the probe 1': the construction is the same as the first embodiment, except as described below.

In this embodiment, the cylindrical portion of the dielectric member 208, is omitted, and the latter includes only the tapering portion 216'. In this embodiment, the material is a dielectric material (Hik 500f or Technox 2000) with permittivity K=25.

The dimensions (in mm) of the probe 1' in this embodiment are as follows.

| a | b | c | d | e | f | g | h | i | j | k |
|---|---|---|---|---|---|---|---|---|---|---|
| 2.7 | 2.3 | 3.5 | n/a | 0.2 | 3 | 2 | 1.1 | 2.9 | 2 | 4.0 |
| n/a - not applicable | | | | | | | | | | |

This embodiment is of a narrower construction and is suited to treatment of narrower anatomical structures.

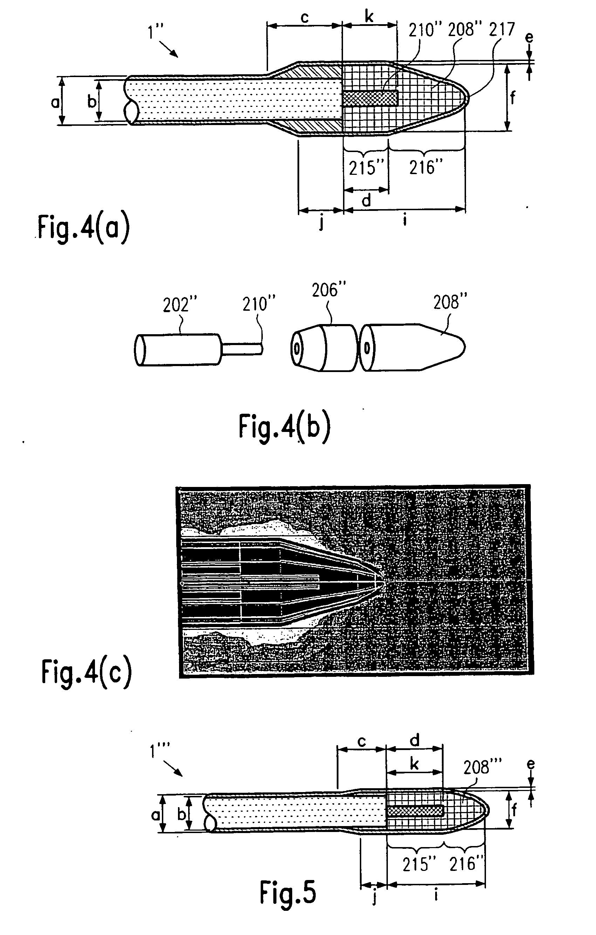

Figure 4 shows views of a third embodiment of the probe 1": the construction is the same as the first embodiment, except as described below.

Referring to Fig. 4(a), in this embodiment, the conductor 210" does not extend along the entire length of the dielectric member 208": here, it extends about half way, and slightly beyond the axial length of the cylindrical portion 215". Also, the tip member is omitted and the tip of the probe 1" is therefore formed by the end tip 217 of the tapering section 216". In this embodiment, the material is a dielectric material (Hik 500f or Technox 2000) with permittivity K=25.

Figure 4(b) is an exploded perspective view of the probe 1 ", showing its main constituent parts. The dimensions (in mm) of the probe 1" in this embodiment are as follows.

| a | b | c | d | e | f | q | h | i | j | k |
|---|---|---|---|---|---|---|---|---|---|---|
| 3.1 | 2.7 | 5 | 3 | 0.2 | 4.4 | n/a | n/a | 8.1 | 3 | 3.5 |
| n/a - not applicable | | | | | | | | | | |

Figure 4(c) is a view of the radiation field pattern generated by the probe 1" of Fig. 4(a) in use. Lighter areas indicate greater intensity: it can be seen that in this embodiment the maximum intensity (white area) is located away from the tip of the probe 1"; rather it is concentrated at the base of the dielectric member 208". This embodiment is designed to deliver heat as uniformly as possible. Assuming that sufficient heat has been delivered, occlusion may be achieved at some point post-treatment.

Figure 5 shows a fourth embodiment of the probe 1"': the construction is the same as the third embodiment, except as described below.

In this embodiment, the dielectric member 208"' is of a smaller, thinner construction. In this embodiment, the material is a dielectric material (Hik 500f or Technox 2000) with permittivity K=25. Here, the conductor 210"' does not extend along the entire length of the dielectric member 208"': it extends by an amount about equal to the axial length of the cylindrical portion 215"'.

The dimensions (in mm) of the probe 1 in this embodiment are as follows.

| a | b | c | d | e | f | g | h | i | j | k |
|---|---|---|---|---|---|---|---|---|---|---|
| 2.7 | 2.3 | 3.5 | 4.1 | 0.2 | 3 | n/a | n/a | 7.1 | 2 | 4.1 |
| n/a - not applicable | | | | | | | | | | |

This embodiment is of a narrower construction and is suited to treatment of narrower anatomical structures.

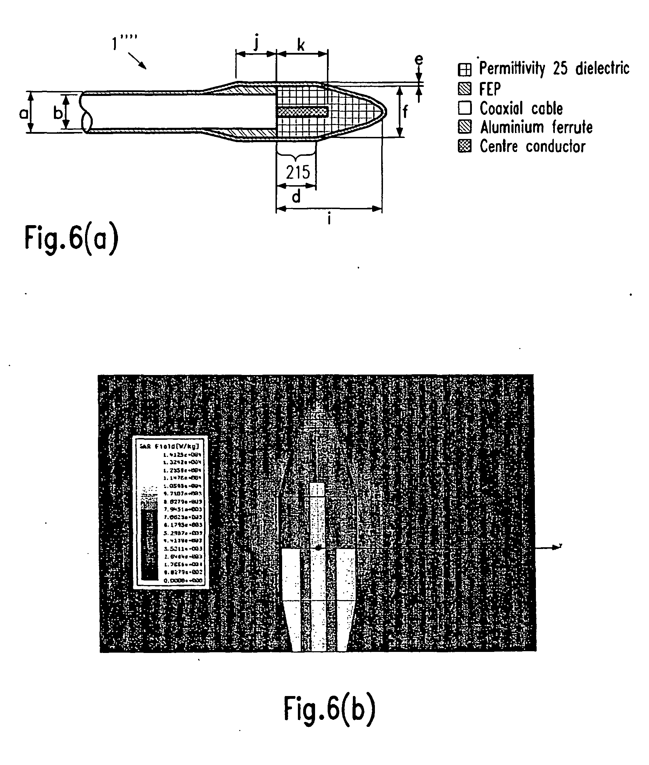

Figure 6(a) shows a cross-sectional view of a fifth embodiment of the probe 1"": the construction is the same as the third embodiment, except as described below.

The dimensions of the probe 1 "" in this embodiment are as follows.

| a | b | c | d | e | f | g | h | i | j | k |
|---|---|---|---|---|---|---|---|---|---|---|
| 3.1 | 2.7 | n/a | 3 | 0.2 | 4.1 | n/a | n/a | 8.1 | 3 | 3.9 |
| n/a = not applicable | | | | | | | | | | |

At a thickness (f) of the dielectric of 4.1, this embodiment is intermediate the third and fourth embodiments in transverse thickness.

Including the sheath 214, the probe 1"" has a diameter of 4.5mm, enabling access via a commercially available percutaneous introducer, well known to persons skilled in the art. Compared with other embodiments, in order to give a smaller burn (via radiation into tissue), for this embodiment the power level is reduced (from 1.4 W per mm of circumference to 1.2 W/mm circumference), and the withdrawal rate increased.

Figure 6(b) shows a plot of the resulting radiation field pattern. Lighter areas indicate greater intensity: it can be seen that in this embodiment the maximum intensity (white area) is a relatively narrow zone extending adjacent to the cylindrical portion 215 of the dielectric. Compared with other embodiments, the withdrawal rate in this case may be in the range 17-20 cm/minute, and is preferably 17.5 cm/minute (rather than 15 cm/minute, as in the other embodiments). An advantage of this embodiment is that less energy per unit length is directed into the tissue, resulting in a reduced burn and a shorter treatment.

As used herein, references to the probe 1 are, as appropriate, references to the probe 1, 1', 1", 1''', and/or 1"". Similarly, references to the dielectric member 208 are, as appropriate, references to the dielectric member 208, 208', 208", 208''', and 208""; and so on.

In all the embodiments described above, the length of the central conductor 210 is selected to optimise the transfer of energy, i.e. to minimise reflection of radiation when the probe 1 is in tissue. The length of the centre conductor is an integer number of quarter wavelengths long, this wavelength being determined by frequency of the radiation and the permittivities of the materials surrounding the centre conductor within the microwave field. Calculating the length of a particular integer number of quarter wavelengths in the dielectric therefore gives an approximate appropriate length. This is then altered and optimised by testing (either on computer or by building prototypes) to take account of the complex interactions of the waves in order to achieve minimum reflected power.

Similarly, in all embodiments, the probe 1 is shaped and dimensioned for ease of use: its rounded form enables the probe 1 to be readily inserted into a vein and also allow the vein to shrink easily back to its original size (diameter) following withdrawal.

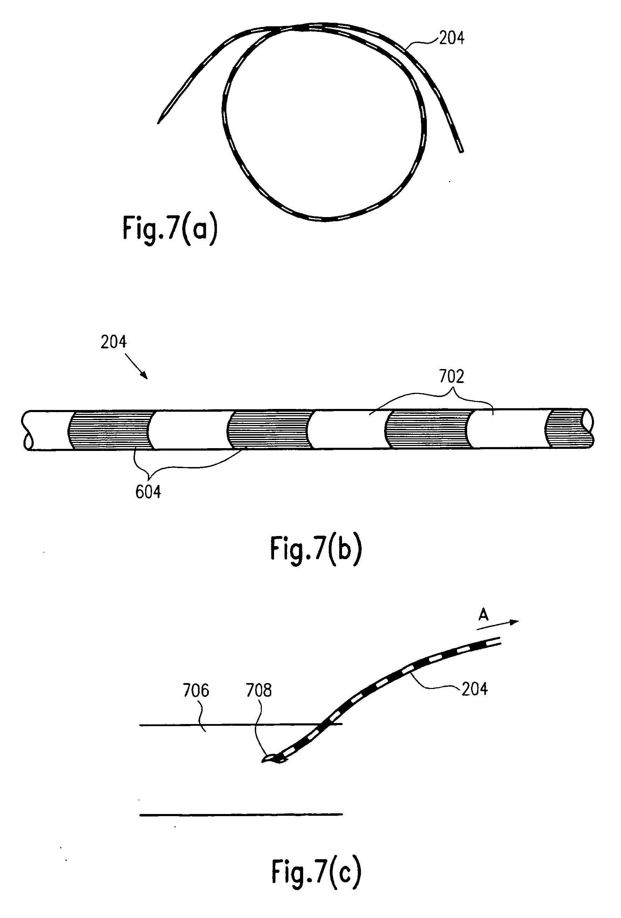

Figure 7 illustrates the coaxial cable 204 employed in the implementation of one aspect of the present invention. More specifically, Fig. 7(a) is a view of the entire cable 204 having the probe 1 fixed to one end thereof, the cable 204 having visible markings thereon.

Referring to Fig. 7(b), this is a close up view of part of the cable 204. In this embodiment, equal length, alternating light sections 702 and dark sections 704 comprise the markings provided on the surface of the cable 204. The markings may be formed during fabrication of the outer casing of the cable 204 itself, or they may be formed by the application of light tape or paint to a dark coloured cable 204, or vice versa, post-manufacture. For example, the markings may be black and white, or black and yellow. Suitably, the markings are about 1 cm wide. The markings provide advantages in use, as further described hereinafter. In other embodiments, the markings may be of unequal length, or they may have a length that varies in a predetermined fashion along the length of the cable 204.

As shown in Fig. 7(c), the probe 1 (not shown) on the end of cable 204 has been inserted into a hollow anatomical structure (e.g. a vein; not shown) inside a body part (e.g. a leg) 706 via a very small incision 708. During treatment, as described in detail below, the cable 204 is pulled by the user (medical professional, practitioner, technician, nurse, etc.) in the direction of arrow A, thereby gradually revealing the markings 702, 704 on the cable 204.

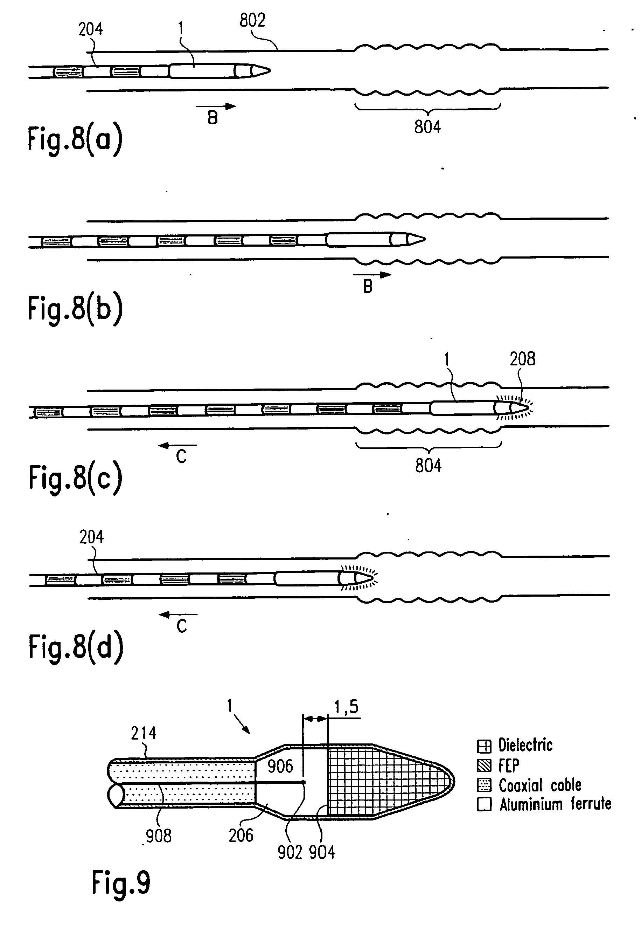

Figure 8 depicts schematically the movement of the probe in the treatment, according to one aspect of the invention, of a varicose vein 802. The diagram is not in proportion: the dimensions and relative size of the vein 802 and probe 1 have been altered merely for the sake of clarity and ease of illustration. The vein 802 will have already been identified and diagnosed as having a section 804 of varicose tissue. Obtaining uniform heating along the length of the vein 802 with a fixed power output is achieved by controlled withdrawal of the probe 1. The rate of withdrawal is correlated to the depth of thermal penetration.

The preferred methodology is as follows.

The maximum size of the vein to be treated (e.g. the greater saphenous vein - GSV) is determined by ultrasound scanning prior to the procedure. A probe with an outer diameter as close as possible to this maximum size is then chosen.

Preferably, a probe 1 is selected with an outer diameter at least as large as the inner diameter of the section of vein to be treated; and this ensures that, during operation, the probe 1 will be tight against, and even expand, the inner wall of the vein, so that the minimum amount of blood is subjected to radiation. This has two further effects: (i) to maximise the microwave energy deposited in the vein wall; and (ii) to evenly treat the whole circumference of the vein wall (as the probe and vein wall are thus concentric). In an alternative procedure, also to minimise the amount of irradiated blood, a measure may be taken to stem the flow of blood through the vein (e.g. "Pringle manoeuvre") so that minimal amounts of blood are subjected to the radiation treatment.

An incision is then made in order to insert the probe at the end of the length of vein 802 to be treated. The probe may be inserted percutaneously, through a catheter, or directly introduced following exteriorisation of the vein 802, as is known in the art. In the case of the GSV, this is likely to be either at the ankle, at the knee, or both.

The probe 1, once it has been introduced into the vein 802 by suitable incision (not shown), is threaded up, or down, i.e. by the user pushing the cable 204 in the direction of arrow B, as shown in Fig. 8(a), to where the treatment is to start. The movement continues, indicated in Fig. 8(b), so that the probe 1 is moved past the varicose section 804 of the vein 802. In the case of the GSV, this means threading the probe up to the saphenofemoral junction.

As illustrated in Fig. 8(c), the emitting part (dielectric member 208) is paused at or just beyond the end of the varicose section 804, the relative positions being determined by suitable means, such as ultrasound scanning. Next, the microwave delivery system (see Fig. 1) is activated. This system is preferably configured, by suitable programming or software tool, so that audible tones (or "beeps") are emitted so as to be easily and distinctly heard by the user. The tones are emitted at a regular rate; however, means may be provided, using techniques well known in the art, for varying the frequency of the beeps between procedures or between patients (i.e. different treatment intensities for different treatments of the same patient at different times (occasions), or different treatment intensities for different patients at different times). Alternatively, the tones may be generated by a conventional tone-generating device separate from the main system.

In another embodiment, the "beeps" may be emitted at a non-regular rate, with the markings on the cable 204 being a uniform pattern with equal length markings, thereby generating a pattern of radiation intensity that varies along the length of the vein section treated.

In another embodiment, the "beeps" may be emitted at a regular rate, with the markings on the cable 204 being a non-uniform pattern with unequal length markings, thereby generating a pattern of radiation intensity that varies along the length of the vein section treated.

In another embodiment, the "beeps" may be emitted at a non-regular rate, with the markings on the cable 204 being a non-uniform pattern with unequal length markings, the pattern of radiation intensity along the length of the vein section accordingly having a different pattern to achieve the desired and designed variable penetration.

It should be noted that evenly irradiating the vein wall is not necessarily the same as evenly heating the vein wall. The probe tip starts at body temperature. When power is first applied some heat is lost to the probe as it heats up. More energy per unit length of vein is therefore required to obtain the same depth of thermal penetration. The withdrawal rate therefore needs to be slower to start with and speed up as the applicator warms up.

The radiation is switched on via the system's user interface, as indicated in Fig. 8(c); this, in turn and via the software causes the emission of "beeps" to commence (the operation of the software is discussed in further detail hereinbelow). Thereupon the user, grasping the cable 204, withdraws the probe 1 by pulling the cable 204 in the direction of arrow C. In doing so, the user ensures that he pulls at such a rate that the markings 702, 704 become visible (Fig. 7 (c)) in succession, one (e.g. black or white) marking in time with each successive tone or "beep". In this way, the radiation emitting dielectric member 208 on the probe 1 passes along the varicose section 804 at a uniform or near-uniform rate while emitting the controlled dose of radiation. When the user achieves this, the thermal penetration of the probe in homogeneous tissue can be effectively guaranteed: this will typically be to a depth of about 1.5mm. This provides for effective treatment of the varicose section via microwave-induced thermal ablation, causing therapeutic occlusion of the tissue.

As the probe comes to the end of the length to be treated (Fig. 8(d)), a red band appears on the shaft to warn the user that the probe is about to appear. At the end of this red band the user turns off the power and thus stops the treatment.

The treatment may, in some cases, be performed in conjunction with ligation.

Referring to Fig. 9, during all operation, the temperature of the tissue is constantly sensed by the thermocouple 902 on the probe 1 and the temperature monitored. Although the withdrawal rate is determined by the audible tones, the system is still required to ensure that the user withdraws while ensuring patients safety. The thermocouple 902 is kept relatively cool due to it constantly being brought into contact with unheated tissue. It is typically 1-2mm back from the ferrule-dielectric interface 904. In the illustrated embodiment (corresponding to the probe design of Fig. 4(a)), the thermocouple 902 is 1.5 mm back from the ferrule-dielectric interface 904. However, this distance may be set at different values, depending on the embodiment of the probe 1. The thermocouple 902 is mounted on the exterior surface 906 of the ferrule 206, suitably by gluing, taping or any other suitable fixing technique. The thermocouple 902 is insulated from the metallic ferrule 206, for example by means of insulating tape. The reading from the thermocouple is carried away via line 908 to the control system (computer) of Fig. 1. As mentioned with respect to Fig. 2, the thermocouple 902 is covered by the protective sheath 214 when the latter is provided over the probe 1.

In the event of the user failing to withdraw, or not withdrawing quickly enough, thermal conduction carries heat forward to the thermocouple. This is used as a safety parameter that can be used to switch the microwave power off. The temperature measured by the thermocouple is typically 60°C. Preferably, the system is configured (e.g. programmed) to cut out the microwave power if the measured temperature reaches 70°C.

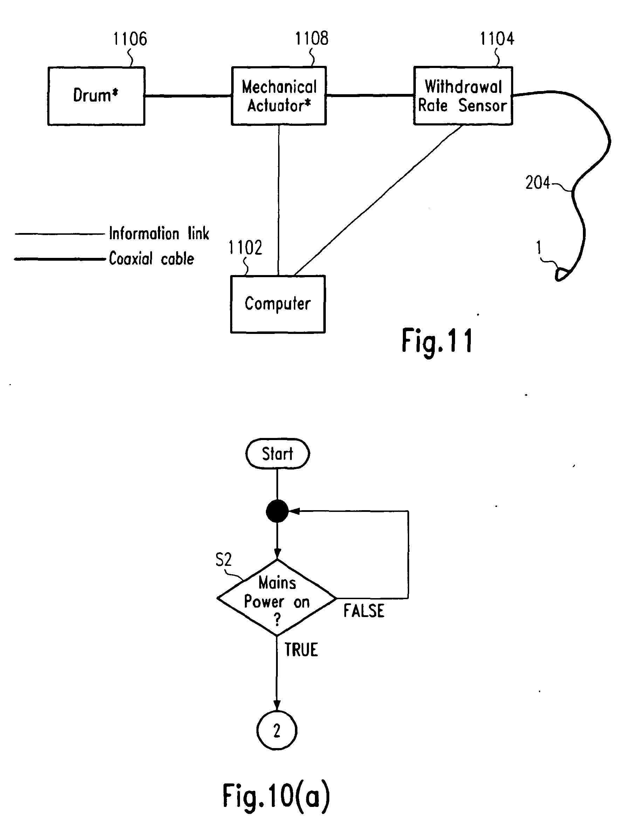

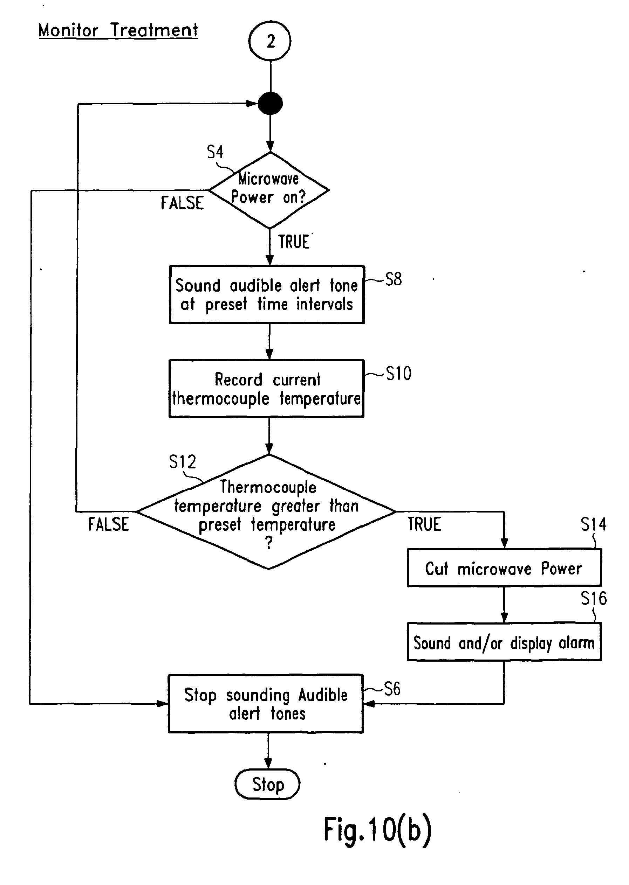

Figure 10 illustrates by flow chart the operation of the software employed on the user's control computer in implementing the invention. As indicated in Fig. 10(a), a check for switch on of mains power to the microwave delivery system is constantly made until it is determined (s2) that this has occurred (TRUE), whereupon processing moves to the next stage.

Further processing commences at ② in Fig. 10(b). Here, a check is made at s4 to see if microwave power to the probe 1 is ON. If it is not ON (i.e. FALSE), either the sounding of audible tone is ceased (at s6) or the sounding is not initiated.

If it is determined at s4 that microwave power to the probe 1 is ON (TRUE), then the sounding of audible tones at predetermined intervals as hereinbefore described is initiated (s8). Next, the current temperature sensed by the thermocouple is recorded. At this stage, a comparison is made (s12) to see if the current thermocouple temperature exceeds a preset threshold temperature. If FALSE (threshold not exceeded), the processing returns to step s4.

If the test is TRUE (temperature exceeds threshold), the microwave power is instantaneously cut at s14, and alarm signal is sounded (different from the series of audible tones) and/or an alert message displayed (s16). Thereafter, processing continues to s6, where the series of audible tones is ceased, and the procedure terminates.

Turning to Fig. 11, this shows arrangement(s) for handling the withdrawal of the cable 204 and probe 1 in alternative embodiments of the invention. The markings combined with the tones provide a simple way to control the withdrawal rate. However, in the embodiments of Fig. 11, computer 1102 is the control computer of Fig. 1, and the cable 204 is withdrawn via a withdrawal rate sensor 1104 linked to computer 1102. Optionally, a drum 1106, for receiving the wound up cable 204, is provided, and this may be via a mechanical actuator 1108 that is also linked to computer 1102.

The operation of various embodiments is as follows.

Various purely mechanical systems may be implemented. In one example, the cable 204 is reeled back onto the drum 1106 whose speed of rotation is predetermined (e.g. driven by a variable speed motor (not shown). In another example, the cable 204 is pulled back through rollers (not shown) whose speed is predetermined (in a similar manner to the driven rollers in sheet feeding apparatus, such as printing and copying machines). Alternatively, the cable 204 is pulled back through the rollers and then reeled onto the drum 1106.

In an embodiment employing manual withdrawal of the cable 204 with feedback guidance, the speed of withdrawal of the cable 204 is sensed through rollers placed on the cable 204, or through which the cable passes, by the drum 1106 reeling the cable 204 in, or by an optical sensor (not shown) detecting the movement of the cable 204 itself.

In an embodiment employing mechanical techniques with sensor feedback, the mechanical withdrawal is monitored by the withdrawal rate sensor 1104, e.g. an optical sensor, in order to detect the movement of the cable 204 and ensure that the correct withdrawal rate is being achieved. The mechanical actuator and drum may or may not be used.

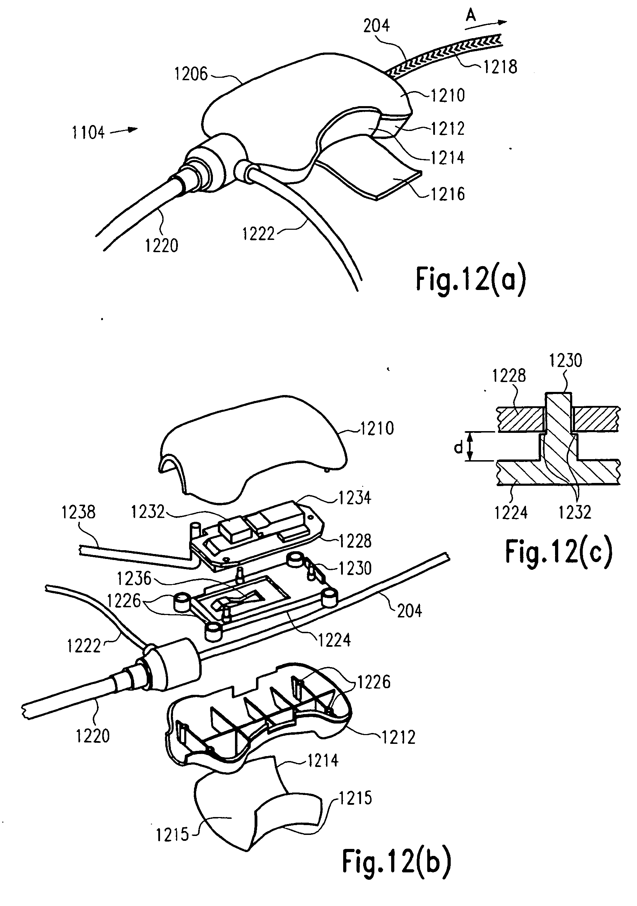

Figure 12(a) shows a close-up perspective view of the motion sensor 1104 of Fig. 11. As can be seen, the housing 1206 is in two parts - an upper housing 1210 and a lower housing 1212, suitably fixed together by screws (not shown). Also, a lower clip assembly 1214 may be attached (by screws, not shown) to the lower housing 1212: the clip assembly 1214 is a generally U-shaped cross-section and includes elongate slots (discussed below) enabling a strap 1216 to pass therethrough; the strap 1216 in turn enables the sensor 1104 to be fixedly and stably attached to another object. In the illustrated example, the strap 1216 can be used to attach the sensor 1104 to the limb of a human body (not shown).

In use, the cable 4 may be pulled in the direction of arrow A, for example by a powered mechanical device such as an electric motor (not shown), by hand, or otherwise. Optionally, the electric motor may be coupled to the same control unit (not shown; discussed further below) that receives signals, as described hereinafter, from the sensor 2 indicative of the rate of movement of the cable 204.

In the illustrated example, the cable 204 comprises coaxial cable having a transparent outer jacket, so that the repetitive pattern of the braided outer conductor of the cable 204 is visible. The detection of the (motion of the) pattern 1218 in this embodiment is discussed in further detail below. However, it will be appreciated that the pattern of this braid need not be repetitive, and the jacket need not be transparent. There just needs to be sufficient surface variation on the surface of the cable 204 that is visible by the sensor 1104 to allow it find recognisable features and so detect relative position. This variation can be so small that it is not visible to the naked eye.. However, in each instance, suitable calibration of the cable (discussed hereinbelow) if preferably employed.

Optionally, in this embodiment, an adaptor 1220, attached to the housing 1206, may be provided, for initial channelling of a device 1 and the cable 204 in a direction opposite to that indicated by arrow A, and for guiding it into housing 1206 during its return travel. An attached tube 1222 may be used to extract fluids.

Figure 12(b) is an exploded perspective view of the motion rate sensor 1104 of Fig. 12(a). The various components of the sensor are shown, including the upper housing 1210, lower housing 1212, and the clip assembly 1214 having two elongate slots 1215 for receiving the strap 1216 (see Fig. 12(a)).

The cable 204 passes generally parallel to the axis of elongation of the housing 1206, and between the lower housing 1212 and a base plate 1224, the latter of which has screw holes 1226 (four of them, in this case) through which fixing screws (not shown) pass from corresponding holes 1226 in the lower housing 1212 upon assembly.

Mounted above the base plate 1224 is a PCB 1228 (which will be described further hereinafter), and projection 1230 (here: three) are provided on the base plate 1224 for this purpose. Referring briefly to the partial cross-section depicted in Fig. 12(c), the projections have two sections of varying diameter, so as to provide shoulders 1232 upon which the PCB 1228 rests, following assembly.

Referring to Fig. 12(b), mounted on the PCB 1228 is a detector device 1232 coupled by serial link to a microcontroller 1234. The detector device 1232 suitably comprises a commercially available optical mouse chip, LED and lens package (ADNK-2620; available from Agilent Technologies); and the microcontroller 1234 suitably comprises a Microchip 1216 series microcontroller (Part. No. PIC16F627). In use, the LED (not shown) of detector device 1232 projects light of a certain wavelength generally downwardly, through optical reader aperture 1236 in base plate 1224; the light is incident on the (repetitive pattern 1218 of the cable 204) and light reflected back off the cable focussed by the lens onto a sensor element of the detector device 1232. From the variation in received optical signal caused by the movement of the pattern 1218 on the cable 204, the detector device 1232 generates corresponding electronic signals that are passed to microcontroller 1234. In turn, the microcontroller 1234 passes signals via cable 1238 (conventional RS-232 interface, for example) to a remote control unit, which is described in more detail hereinafter.

It is to be noted that the accuracy of the measurement by the optical motion sensor 1104 is largely determined by the degree of play between the cable 204 and the lens. Some form of channel, holder or guide surface, used in a preferred embodiment, to guide the cable 204 in place under the lens is therefore important. The greater the vertical motion of the cable 204 in this guide, the less accurate the motion rate measurement will be.

It will be appreciated that each of the components 1210, 1212, 1214, 1224, 1228 of the sensor 1104 may be made of conventional materials (e.g. plastics) using well-known moulding techniques. And, when assembled, the sensor 1104 may be of compact dimensions and may be in a form similar to a conventional computer mouse.

In an alternative embodiment, magnetic sensing of the rate of motion is used. Here, magnets are placed in or on the cable. Two possible ways of detecting the magnetic fields may be employed. In a first technique, the cable passes through a coil of wire that is built into the housing. As the magnets in the cable pass through coil they generate a current (pulse) that is detected. Either the rate of generation of these pulses, or the magnitude of each pulse, allows calculation of the rate of motion. In a second technique, a Hall probe is used to measure the magnetic field. Once again, the rate of detection of the pulses of magnetic field in conjunction with knowledge of the spacing between magnets, allows the rate of movement to be calculated.

In a further alternative embodiment, reflector elements for sensing of the rate of motion are used. Instead of the Agilent chip described above, a photodiode or phototransistor is used in conjunction with a cable that incorporates more and less reflective sections. Bands alternately having higher and lower reflectivity are used. As each more reflective band passes the sensor, the degree of light detected by the photo detector increases. Once again, knowing the spacing of the bands and the rate of detected pulses allows calculation of the rate of movement.

In a further alternative embodiment, radioactivity detection for sensing of the rate of motion is used. Here, radioactive particles are regularly spaced on the cable. A radioactivity sensor (such as that used in smoke alarms) is used to detect the resulting radiation and provide a pulse as each particle passed the sensor. Once again, knowledge of the spacing between particle and the rate of pulses allows calculation of the speed.

In a further alternative embodiment, resistance measurement for sensing of the rate of motion is used. Alternatively, the article has regions of varying dielectric strength and passes through the plates of a capacitor. Alternatively, the article has regions of varying thickness and a proximity measurement is made. Alternatively, the article has regions of opacity and transparency, with a source and detector facing each other with the article passing between the two. (This opacity and transparency could be to visible light, any electromagnetic signal, radioactivity, magnetic field, or electric field.)

All of these alternative embodiments may be used in conjunction with the example provided above that employs the Agilent LED/optical sensor chip, to provide additional information on the position of the cable. For example, a small reflective band, or magnetic particle may be placed near the end of the cable. If the housing incorporates the relevant detection device, when the cable reaches this position, a single pulse will be delivered to the system to signal that the end of the cable is approaching.

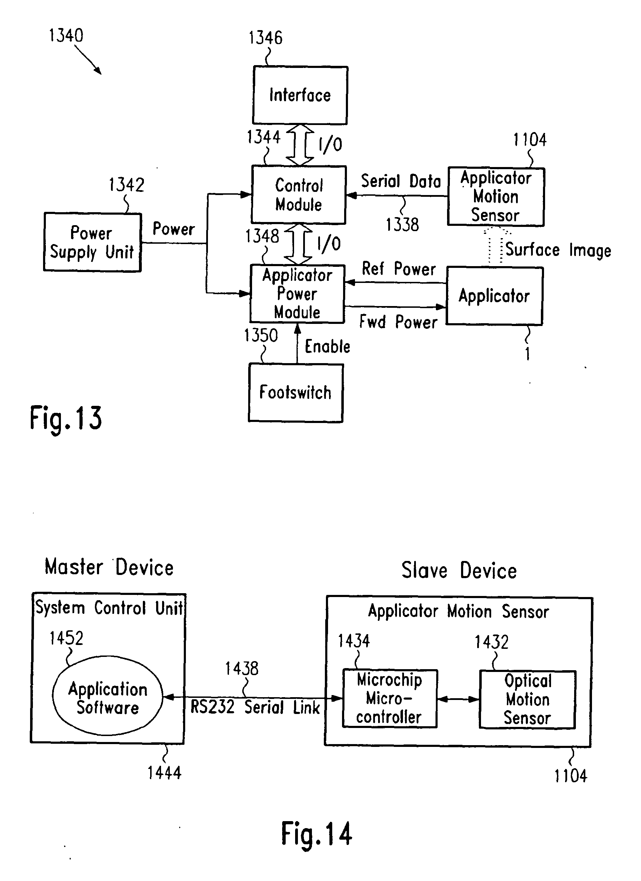

Figure 13 schematically illustrates a system, in accordance with one aspect of the invention, for performing the controlled movement of an article, using the above-described motion rate sensor 1104. The system (generally designated 1340) includes a PSU 1342, a control module 1344 and a user interface (UI) 1346. As mentioned, the motion rate sensor 1104 is coupled to the control module 1344 by serial link (1338). As is conventional, the UI 1346 may present graphical, audible or graphical and audible information (not shown) to the user via well-known display and/or speaker technology, under the control of the control module 1344.

As mentioned with reference to Fig. 11, in the illustrated embodiment, the cable 204 may be attached to a medical device 1 (treatment applicator) whose rate of motion is to be monitored/controlled. Optionally, therefore, with reference to Fig. 13, the system 1340 may include the medical device 1 (applicator), a power module 1348 able to adjust power supplied to the medical device 1 in dependence upon the motion rate data supplied to it by the control-module, and a user-operable footswitch 1350 enabling the user to switch the power on or off.

Figure 14 illustrates schematically in more detail the communication between the motion rate sensor 1104 and the control module 1344 in Fig. 13. When the sensor 1104 is connected to the system 1440 to provide feedback to the user, the data is suitably transferred by a standard protocol, such as RS232. The sensor 1104 is connected to the control unit 1444 of the system 1440, and the comms port (not shown) of the system 1440 regularly polled by the system's software 1452 to extract the data on the sensor's position. From the position data, the rate of motion can be derived. However, it will be appreciated that other protocols (and cables) may be used instead of RS232, e.g. RS485, RS422, 12C, USB, GP1B, parallel or other protocol. The rate of polling the sensor 1104 to determine the motion rate is determined by the degree of resolution of the sensor and the desired motion rate (as discussed hereinafter).

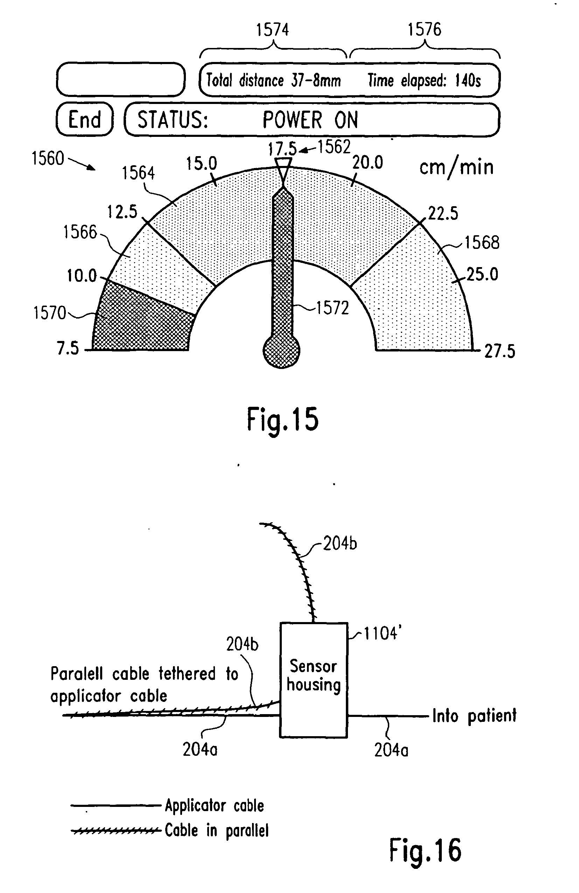

Figure 15 depicts an example of a UI view displayed to the user by the system 1340 of Fig. 13. As can be seen, there is displayed graphically a "speedometer-type" meter 1560. The meter 1560, as well as displaying specific values, including the current value 1562, also has several coloured zones, including a green zone 1564, two orange zones 1566, 1568, and a red zone 1570. Thus, the user has visual feedback as to whether the rate of motion of the article 1 sensed by the sensor 1104 (see Fig. 1)

  (i) is at an optimal value (pointer 1571 pointing straight up),
  (ii) is at an acceptable value (pointer 1572 within green zone 1564),
  (iii) is at a somewhat unacceptable value (pointer 1572 within orange zones 1566, 1568), or
  (iv) is at a highly unacceptable value (pointer 1572 within red zone 1570).

Alternatively or additionally, audible information may be emitted by the UI 1546, corresponding to the different aforementioned zones, i.e. with green->orange->red zones corresponding to increasingly higher tone (sonic frequency), or corresponding to sonic pulses ("beeps") being emitted at different (increasing green->orange->red) rate for different zones.

Additionally, the UI 1346 may be caused by software to display (at 1574) the total distance travelled by the article (cable) 204, and/or (at 1576) the total elapsed time during the travel of the article.

Figure 16 shows an alternative embodiment of the motion rate sensor 1104'- in the case where two cables are used. The construction is the same as the embodiment of Figs 11 and 12, except as follows. Here, two tethered cables 204a, 204b, that run in parallel for most of their length, separate first before passing through the sensor 1104'. The applicator cable 204a (e.g. supplying power to a treatment device (not shown)) passes through the sensor 1104' transversely. The parallel cable 204b, having detectable markings thereon, passes through the sensor 1104', has its marking detected by the detector device (see Fig. 12), and exits the sensor 1104' longitudinally.

Although the motion rate sensor has been described above in relation to a simple medical application, it will be appreciated by persons skilled in the art that the invention may be employed in any situation in which the rate of movement of an article is to be measured and/or controlled. Examples include all sorts of cable operated devices, equipment and machinery. The forementioned (automatic) cable operated curtains and garage doors are typical examples. The motion of moving rods, cables and threads in manufacturing environments (e.g. the weaving industry) may also be measured using techniques according to the invention.

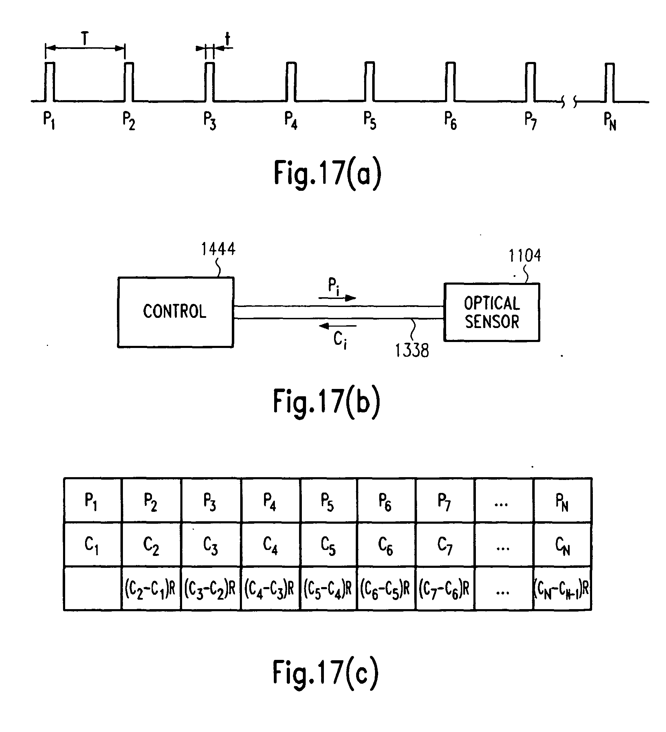

Figure 17(a) illustrates schematically a time sequence of polling signals issued by the control unit 1444 down serial line 1338 to the sensor 1104 (see Fig. 7(b)). As can be seen, the polling (here illustrated schematically as a single pulse of duration t) occurs at regular intervals with a polling interval T. The polling pulses are labelled $p_1$, $p_2$, $p_3$,... $p_N$.

Figure 17(b) shows the transmission of signals between sensor 1104 and control unit 1444, such that, in response to pulse $p_i$, a count $c_i$ is returned by the sensor 2. Suitably, the count $c_i$ is defined by a multi-bit digital signal sent down the serial line 1338. Before use, the sensor 1104 is reset. During use the sensor 1104 need not be re-set. In one embodiment, the polling interval T is 0.2 seconds.

Figure 17(c) shows values obtained in series as a result of the polling. In the first row is the sequence of polling pulses $p_i$, and in the second are the counts $c_i$ that are returned in response to the respective polling pulse. In order to convert these values $c_i$ to values representing motion, the counts value from the time the sensor 1104 was last polled is subtracted from the present counts value. This gives the number of counts the cable 204 has moved relative to the sensor 1104 during the polling interval T. The number of counts is then divided (or multiplied) by a constant (counts-per-inch) to give the actual distance moved in the units required by the system (control unit 1444). It is then divided by the time interval to give the rate of motion. Thus, the speed, i.e. the average speed over the last polling interval T, for a given polling pulse i is given in the third row in Fig. 17(c): speed $v = (c_i - c_{i-1})R$, where R is a conversion factor. If it has been previously determined, e.g. using suitable calibration techniques, discussed hereinafter, that the number of counts-per-inch for the cable is K, then $R = 1/(KT)$. The speed v (in inches/second) is calculated by the control unit 44 using this relation, and using a stored value for R.

In the embodiment described, K is around 460 counts per inch, and T = 0.2 s. So, for example, if the cable moves 10 counts in 0.2 seconds, the rate is (10 / 460) / 0.2 = 0.1087 inches per second. As mentioned, this gives a value of speed v every 0.2 secohds. This is not an instantaneous speed, but a value that is the average speed over the previous 0.2 s

The polling interval T is also important in determining accuracy. It is related to the speed of motion of the cable 204. If T is so short that very few counts have been accumulated between polls, then discretisation errors will occur, as measured rate will appear to be one of several discrete values. The polling interval may need (depending on whether such errors are important in the application) to be chosen such that sufficient counts are read by the sensor 2 between polling pulses to make such errors unrecognisable. A faster cable movement will allow a higher rate of polling (and therefore shorter T). A slower one will require a lower rate of polling. On the other hand, if T is made too long, then the rate recorded will start to noticeably lag behind the actual movement, as it is effectively an average over the polling interval T. In the currently preferred embodiment, the lowest effective value of the polling interval T, with a pullback rate (i.e. rate of movement of cable 4) of 10cm/min or greater, is around 0.2 s.

In an alternative embodiment, if an average speed is required, but without large T and hence long intervals between speed updates, then shorter polling intervals T can be used in conjunction with a rolling average calculation: here, control unit 1444 computes the mean value of the preceding n speed results. This is has the advantage that the displayed or measured speed will vary more smoothly over time. The shorter the polling interval T, the less jerky the response. The greater n is, the more damped the response. This produces advantages in the display of speed to the user, particularly when the aforementioned "speedometer-type" display of cable speed (see Fig. 15) is employed. The number n of past speed values that are averaged may be any suitable number, e.g. from a few to a few tens of values. Typically, $n \leq 32$. In a preferred embodiment, n is 16 (i.e. providing a speed averaged over 16 x 0.2 = 3.2 s). However, it will be appreciated that, depending on the application, the upper limit on n may be several tens, several hundreds or more. The upper limit may have any suitable value reasonably applicable to the parameters of the apparatus being used.

The number of counts measured per unit distance will vary slightly with withdrawal speed. A higher withdrawal speed will result in a slightly lower number of counts per unit distance. In order to assess the appropriate calibration factor to use in the system to convert the number of counts to distance and hence speed, a device has been designed and built to accurately withdraw the cable through the sensor at a known fixed rate. This therefore means that the calibration factor for any particular withdrawal rate can be accurately assessed.

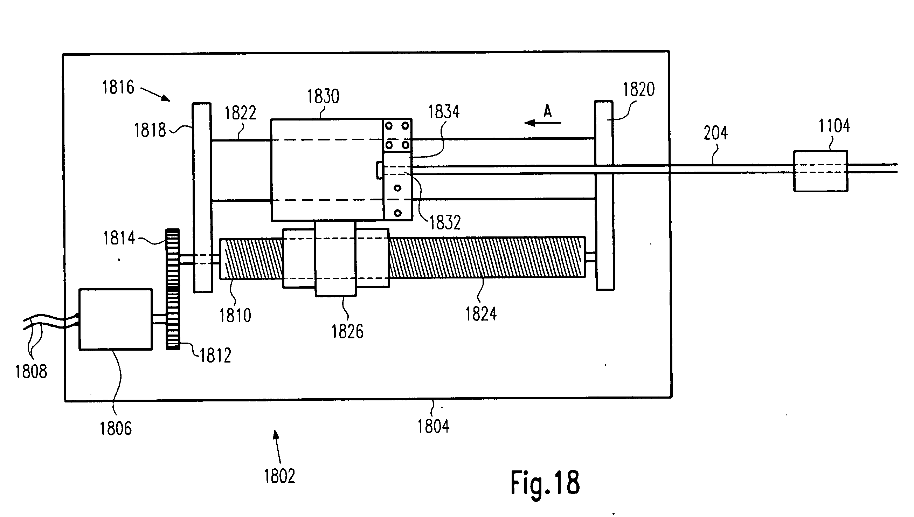

Figure 18 illustrates a plan view of a calibration apparatus 1802 that may be employed according to embodiments of one aspect of the invention. The calibration apparatus 1802 includes a rigid base 1804 onto which various components, described hereinafter, are fixedly mounted. These include a stepper motor 1806 is coupled to a computer (not shown) via cables 1808, and driven under the control of the computer, as is well known in the art. The stepper motor 1806 transmits rotary motion to a lead screw 1810 via co-operating gears 1812, 1814, of the spur gear type. The lead screw 1810 is mounted for rotation on a rigid frame 1816 that includes endplates 1818, 1820, and slide 1822.

The lead screw 1810 is provided with an external helical thread 1824 of constant, known pitch; and a drive nut 1826 is provided on the lead screw 1810 and has a co-operating internal thread (not shown). The drive nut 1826 is in turn rigidly attached to a runner 1830 mounted on the slide 1822. The runner 1830 and slide 1822 preferably have co-operating guide elements and/or wheels/bearings (not shown) so as to facilitate low-friction sliding of the runner 1830 along the slide 1822. Suitably, the length of travel of the runner 1830 on the slide 1822 is of the order of 0.5-1.0m.

A sensor 1104 to be calibrated is provided a suitable distance (e.g. 1-2 m) from end plate 1820 of the frame 1816 and is fixed relative thereto, e.g. by clamping or screws (not shown). The cable 204 passes through the sensor 1104 and its end 1832 is clamped (e.g. by suitable plates and screws) by a clamping element 1834 on the runner 1830.

Calibration (for determining the counts-per-inch K of the cable 204 using the sensor 1104) consists of the following. The sensor 1104 is set up for issuing the accumulated count value $c_i$ after successive polling intervals T. The drive nut 1826 is placed at the end of its travel adjacent the end plate 1820 of frame 1816. With the cable 204 passing through the sensor 1104, and with any excessive slack in the cable 204 removed, the stepper motor 1806 is powered up and driven at constant rotational speed by the control computer (not shown); thus, the runner 1830 is driven along the slide by drive nut 1826 at a known constant linear speed V (inch/s).

A calibration operation may comprise measuring the count at the end of the travel of the runner 1830 and that at the start of the travel. More preferably, a calibration operation comprises measuring the count at least at a "start of calibration", i.e. a predetermined time after the runner has started its motion on the slide, and has clearly reached the constant speed, and measuring the count at the "end point of the calibration", i.e. a predetermined time before the runner has ended its motion. This has the benefit of eliminating distortion due to acceleration and deceleration periods.

If the difference between the count at the end of the travel of the runner 1830 and that at the start of the travel is $C_c$, and the number of polling intervals T is $n_c$, than the counts-per-inch K can be obtained from

$$V = (C_c/K)/ n_c T$$

or

$$K = C_c/n_c TV$$

This is one calibration operation. It will be appreciated that, for one sensor-cable combination, this operation may be repeated several or many times, and the obtained values K averaged.

Once the value K is known, this can be used to measure motion rate, as discussed above in relation to Fig. 17. It will be appreciated by persons skilled in the art that, once a value for K has been established, one or more of the other parameters in the relationship $K = C_c/n_c TV$ can thereafter be obtained.

It will be immediately apparent that, although inches have been used as units in certain embodiments, other units may be used. That is, instead of expressing K in counts-per-inch, this may be counts-per-mm, per-cm, per-m, or counts per custom unit; and instead of expressing V in inches/second, this may be in mm/s, cm/s, m/s; and so on.

**Claims**

1.   A system for the treatment of hollow anatomical structures, the system comprising an applicator (1) for applying radiation to hollow anatomical structures, the applicator (1) comprising:

  an elongate member which includes an emitter, the emitter being coupled to a source of microwave radiation and being adapted to emit said radiation, and the emitter including:

    a radiation emitting portion (208) made of dielectric material and having an axis of elongation, the radiation emitting portion (208) being shaped and dimensioned so as to emit said radiation at a predetermined intensity in a field of limited dimensions adjacent thereto, whereby occlusion of the tissue of a hollow anatomical structure within said field is effectively accomplished, and
    an elongate conductor (210) within and extending at least partially

  along the radiation emitting portion (208).

  **characterised in that** the system further comprises:

    a motion rate sensor (1104) arranged, in use, for detecting the rate of movement of the applicator (1); and,
    a control unit (14, 1102, 1344 1444) coupled to the sensor (1104) for receiving the motion rate signals output thereby,
    wherein the control unit (14, 1102, 1344 1444) is configured to calculate the speed of motion of the applicator (1) using said motion rate signals, and control the amount of radiation supplied to the applicator (1) and/or the rate of motion of the applicator (1) in dependence upon said calculated speed of motion.

2.   The system of claim 1, wherein the radiation emitting portion (208) includes a generally conical tapering portion (216), the tapering portion (216) thereby forming a tip (217) for insertion into a hollow anatomical structure.

3.   The system of claim 2, wherein the elongate conductor (210) extends along the entire length of the radiation emitting portion (208), whereby said field is disposed, in use, substantially around said tip (217).

4.   The system of claim 2, wherein the elongate conductor (210) extends partially along the length of the radiation emitting portion (208), whereby said field is disposed, in use, substantially around the midsection of said radiation emitting portion (208) and spaced apart from said tip (217).

5.   The system of any preceding claim, wherein a temperature sensor (902) is provided on said elongate member, said temperature sensor (902) preferably comprising a thermocouple or a fibre optic sensor.

6. The system of any of claims 2 to 5, wherein radiation emitting portion (208) includes a substantially cylindrical portion (215) integral with the tapering portion (216).

7. The system of any preceding claim, wherein, for calculating the speed of motion of the applicator (1), the control unit (14, 1102, 1344 1444) is configured to poll the motion rate sensor (1104), the polling interval between successive polls being of uniform duration; determine a difference value, the difference value being a difference between counts defined by successive motion rate signals; using the determined difference value and a conversion factor R, calculating the speed of motion of the applicator (1) using R and the difference value.

8. The system of any preceding claim, wherein the speed of motion is calculated using v= (cj-cj,) R where (ci-ci-1) is the difference value.

9. The system of any preceding claim, wherein the applicator (1) is mounted on the end of an elongate cable (204), and the speed of motion of the applicator (1) is calculated by calculating the speed of motion of the cable (204).

10. The system of claim 9, wherein the polling interval between successive polls is T, and the conversion factor is determined as R = 1/KT, where K is a predetermined count conversion constant for the cable (204).

11. The system of claim 5 as appended to claim 9 or claim 10, wherein the cable (204) is a coaxial cable, and a portion of said cable (204) in abutment with the radiation emitting portion (208) is surrounded by, and attached thereto, by a conductive ferrule (206); and wherein the temperature sensor (902) is disposed on the ferrule (206).

12. The system of claim 11, wherein a series of regularly spaced markings (1218) are provided on the exterior surface of the coaxial cable (204) along its length.

13. The system of claim 11, wherein the markings (1218) are non-regularly spaced instead of regularly spaced.

14. The system of claim 12 or claim 13, wherein the markings (1218) comprise alternately light (702) and dark (704) coloured sections.

15. The system of claim 14, wherein the light (702) and dark (704) coloured sections are each about 1 cm long.

16. The system of any preceding claim, wherein the system further includes a display device (20) adapted to display, under the control of the control unit (14, 1102, 1344 1444), the calculated speed of motion of the applicator (1).

17. The system of claim 16, wherein the display device (20) is adapted to display, under the control of the control unit (14, 1102, 1344 1444), a graphical representation (1560) of the calculated speed of motion of the applicator (1).

18. The system of claim 17, wherein said graphical representation (1560) comprises a speedometer- like graphical representation.

19. The system of any of claims 9 to 18, wherein the motion rate sensor (1104) comprises a housing (1206) relative to which, in use, the cable (204) moves, a detection unit (1232) disposed within the housing (1206), the detection unit (1232) including a conversion device adapted for generating detector signals caused by the motion of the article, and processing circuitry (1234) adapted for receiving said detector signals and outputting motion signals indicative of the rate of movement of the article.

20. The system of claim 19, wherein the housing (1206) includes at least one aperture permitting motion of the cable (204) relative to the housing (1206).

21. The system of claim 20, wherein the housing (1206) has a configuration whereby, in use, the movement of the cable (204) in or near the housing (1206) is substantially linear.

22. The system of claim 20 or 21, wherein said at least one aperture includes an entry aperture through which, in use, the cable (204) enters the housing (1206), and an exit aperture through which, in use, the cable (204) exits the housing (1206), the cable (204) preferably moving, in use, in a substantially linear path between said entry aperture and exit aperture.

23. The system of any of claims 19 to 22, wherein the conversion device comprises of at least one radiation detector adapted for receiving radiation from the cable (204) and generating detector signals in dependence on said received radiation.

24. The system of claim 23, wherein the radiation is optical radiation, the detection unit (1232) further includes an optical emitter for emitting the optical radiation, and the radiation detector is disposed so as to receive said optical radiation after reflection from the cable (204).

25. The system of claim 24, wherein the optical emitter is a LED, and preferably wherein the optical emitter and radiation detector comprise an integral device.

26. The system of claim 23, wherein the radiation detector comprises a detector of low-level radioactivity, and the cable (204) has a plurality of radioactive elements disposed therein or thereon in a repetitive pattern along its length.

27. The system of any of claims 19 to 22, wherein the conversion unit includes a magnetic detector, and the cable (204) has a plurality of magnetic elements disposed therein or thereon in a repetitive pattern along its length, the magnetic detector being adapted to generate said detector signals when the cable (204), in use, moves past the magnetic detector.

28. The system of any of claims 19 to 22, wherein the conversion unit includes one or more rotatable members, such as one or more wheels or balls, adapted to contact the cable (204) and be rotated thereby, in use, and an electromechanical device adapted to generate said detector signals in dependence upon the rate of rotation of said rotatable member (s).

**Patentansprüche**

1. System zur Behandlung von hohlen anatomischen Strukturen bzw. anatomischen Hohlräumen, wobei das System einen Applikator (1) umfasst, zum Einsatz von Strahlung an hohlen anatomischen Strukturen, wobei der Applikator (1) umfasst:

ein längliches Bauteil, welches einen Emitter aufweist, wobei der Emitter an eine Mikrowellenstrahlungsquelle gekoppelt ist und angepasst ist, um die Strahlung zu emittieren, und wobei der Emitter umfasst:

eine Strahlung emittierenden Abschnitt (208), hergestellt aus dielektrischem Material, und mit einer länglichen Achse, wobei der Strahlung emittierende Abschnitt (208) so geformt und dimensioniert ist, dass er die Strahlung mit einer vorbestimmten Intensität in einem begrenzten benachbarten Feld emittiert, wobei die Okklusion des Gewebes der hohlen anatomischen Struktur im Feld wirksam erreicht wird, und einen länglichen Leiter (210) innerhalb des Strahlung emittierenden

Abschnitts (208) und zumindest teilweise darüber hinausreichend,

**dadurch gekennzeichnet, dass** das System weiterhin umfasst:

ein Bewegungssensor (1104), der während der Verwendung so angeordnet ist, dass er die Bewegung des Applikators (1) detektiert bzw. aufnimmt; und
eine Steuerungs- bzw. Kontrolleinheit (14, 1102, 1344, 1444), gekoppelt an den Sensor (1104), um den Bewegungssignaloutput hiervon zu empfangen,
wobei die Steuerungs- bzw. Kontrolleinheit (14, 1102, 1344, 1444) konfiguriert ist, um die Bewegungsgeschwindigkeit des Applikators (1) unter Verwendung der Bewegungssignale zu berechnen, und die vom Applikator (1) gelieferte Strahlungsmenge und/oder die Bewegung des Applikators (1) in Abhängigkeit von der berechneten Bewegungsgeschwindigkeit zu steuern bzw. zu kontrollieren.

2. System nach Anspruch 1, wobei der Strahlung emittierende Abschnitt (208) einen im allgemeinen konischen, sich verjüngenden, Abschnitt (216) aufweist, wobei der sich verjüngende Abschnitt (216) somit eine Spitze (217) zum Einführen in eine hohle anatomische Struktur bildet.

3. System nach Anspruch 2, wobei sich der längliche Leiter (210) über die gesamte Länge des Strahlung emittierenden Abschnitts (208) erstreckt, wobei das Feld während der Verwendung im Wesentlichen um die Spitze (217) angeordnet ist.

4.  System nach Anspruch 2, wobei sich der längliche Leiter (210) teilweise entlang der Länge des Strahlung emittierenden Abschnitts (208) erstreckt, wobei das Feld während der Verwendung im Wesentlichen um den mittleren Bereich des Strahlung emittierenden Abschnitts (208) und von der Spitze (217) beabstandet angeordnet ist.

5.  System nach irgendeinem der vorhergehenden Ansprüche, wobei ein Temperatursensor (902) auf dem länglichen Bauteil vorgesehen ist, wobei der Temperatursensor (902) bevorzugt ein Thermoelement oder einen Faseroptiksensor umfasst.

6.  System nach irgendeinem der Ansprüche 2 bis 5, wobei der Strahlung emittierende Abschnitt (208) einen im Wesentlichen zylindrischen Abschnitt (215) umfasst, der einstückig mit dem sich verjüngenden Abschnitt (216) vorliegt.

7.  System nach irgendeinem der vorhergehenden Ansprüche, wobei für die Berechnung der Bewegungsgeschwindigkeit des Applikators (1) die Kontroll- bzw. Steuereinheit (14, 1102, 1344, 1444) konfiguriert ist, um den Bewegungssensor (1104) abzufragen, wobei das Abfrageintervall zwischen aufeinanderfolgenden Abfragen von gleicher Dauer ist; den Differenzwert bestimmt, wobei der Differenzwert eine Differenz zwischen Werten darstellt, definiert durch aufeinanderfolgende Bewegungssignale; Verwenden des bestimmten Differenzwertes und einem Umwandlungsfaktor R, Berechnen der Bewegungsgeschwindigkeit des Applikators (1) unter Verwendung von R und dem Differenzwert.

8.  System nach irgendeinem der vorhergehenden Ansprüche, wobei die Bewegungsgeschwindigkeit berechnet wird unter Verwendung von $v = (c_j - c_j,) R$, wobei $(c_i - c_{i-1})$ der Differenzwert ist.

9.  System nach irgendeinem der vorhergehenden Ansprüche, wobei der Applikator (1) am Ende eines langen Kabels (204) angebracht ist, und die Bewegungsgeschwindigkeit des Applikators (1) durch die Berechnung der Bewegungsgeschwindigkeit des Kabels (204) berechnet wird.

10. System nach Anspruch 9, wobei das Abfrageintervall zwischen aufeinanderfolgenden Abfragen T darstellt und der Umwandlungsfaktor bestimmt wird als $R = 1/KT$, wobei K eine vorbestimmte Zählwertumwandlungskonstante für das Kabel (204) darstellt.

11. System nach Anspruch 5, wie rückbezogen auf Anspruch 9 oder Anspruch 10, wobei das Kabel (204) ein Koaxialkabel darstellt, und ein Teil des Kabels (204), anliegend an den Strahlung emittierenden Abschnitt (208), umgeben und daran befestigt ist mit einer leitenden Hülse bzw. Ferrule (206); und wobei der Temperatursensor (902) auf der Hülse bzw. Ferrule (206) angeordnet ist.

12. System nach Anspruch 11, wobei eine Reihe von regelmäßig beabstandeten Markierungen (1218) auf der äußeren Oberfläche des Koaxialkabels (204) entlang seiner Länge angebracht sind.

13. System nach Anspruch 11, wobei die Markierungen (1218) anstatt gleichmäßig beabstandet ungleichmäßig beabstandet sind.

14. System nach Anspruch 12 oder Anspruch 13, wobei die Markierungen (1218) abwechselnd hell (702) und dunkel (704) gefärbte Abschnitte aufweisen.

15. System nach Anspruch 14, wobei die hell (702) und dunkel (704) gefärbten Abschnitte jeweils etwa 1 cm lang sind.

16. System nach irgendeinem der vorhergehenden Ansprüche, wobei das System weiterhin umfasst: eine Anzeigevorrichtung (20), angepasst, um, gesteuert durch die Kontroll- bzw. Steuereinheit (14, 1102, 1344, 1444), die berechnete Bewegungsgeschwindigkeit des Applikators (1) anzuzeigen.

17. System nach Anspruch 16, wobei die Anzeigevorrichtung (20) angepasst ist, um, gesteuert durch die Kontroll- bzw. Steuereinheit (14, 1102, 1344, 1444), eine graphische Darstellung (1560) der berechneten Bewegungsgeschwindigkeit des Applikators (1) anzuzeigen.

18. System nach Anspruch 17, wobei die graphische Darstellung (1560) eine Tachometerähnliche graphische Darstellung aufweist.

**19.** System nach irgendeinem der Ansprüche 9 bis 18, wobei der Bewegungssensor (1104) ein Gehäuse (1206) aufweist, relativ zu dem sich das Kabel (204) bei Verwendung bewegt, eine Bestimmungseinheit (1232), angeordnet innerhalb des Gehäuses (1206), wobei die Bestimmungseinheit (1232) eine Umwandlungsvorrichtung aufweist, die angepasst ist, um Detektorsignale zu erzeugen, ausgelöst durch die Bewegung des Gegenstands, und Verarbeitungsschaltkreise (1234), angepasst, um die Detektorsignale aufzunehmen und Bewegungssignale auszugeben, die die Bewegung des Gegenstands anzeigen.

**20.** System nach Anspruch 19, wobei das Gehäuse (1206) mindestens eine Öffnung aufweist, die die Bewegung des Kabels (204) relativ zum Gehäuse (1206) ermöglicht.

**21.** System nach Anspruch 20, wobei das Gehäuse (1206) eine Konfiguration aufweist, durch die während der Verwendung die Bewegung des Kabels (204) in oder nahe beim Gehäuse (1206) im Wesentlichen geradlinig verläuft.

**22.** System nach Anspruch 20 oder 21, wobei die mindestens eine Öffnung eine Eingangsöffnung umfasst, durch die während der Verwendung das Kabel (204) in das Gehäuse (1206) eintritt und eine Austrittsöffnung, durch die während der Verwendung das Kabel (204) das Gehäuse (1206) verlässt, wobei sich das Kabel (204) während der Verwendung bevorzugt entlang eines im Wesentlichen geradlinigen Pfads zwischen der Eintritts- und der Austrittsöffnung bewegt.

**23.** System nach irgendeinem der Ansprüche 19 bis 22, wobei die Umwandlungsvorrichtung mindestens einen Strahlungsdetektor umfasst, der angepasst ist, um Strahlung vom Kabel (204) aufzunehmen und Detektorsignale in Abhängigkeit von der aufgenommenen Strahlung zu erzeugen.

**24.** System nach Anspruch 23, wobei die Strahlung optische Strahlung darstellt, wobei die Detektionseinheit (1232) weiterhin einen optischen Emitter zum Emittieren optischer Strahlung umfasst, und der Strahlungsdetektor so angeordnet ist, dass er die optische Strahlung nach der Reflektion vom Kabel (204) aufnimmt.

**25.** System nach Anspruch 24, wobei der optische Emitter eine LED darstellt, und wobei der optische Emitter und der Strahlungsdetektor bevorzugt eine einstückige Vorrichtung darstellen.

**26.** System nach Anspruch 23, wobei der Strahlungsdetektor einen Detektor für Radioaktivität auf niedrigem Nievau umfasst, und das Kabel (204) eine Vielzahl von radioaktiven Elementen aufweist, die entlang der Länge darin oder darauf in einem sich wiederholenden Muster angeordnet sind.

**27.** System nach irgendeinem der Ansprüche 19 bis 22, wobei die Umwandlungseinheit einen Magnetdetektor umfasst und das Kabel (204) eine Vielzahl magnetischer Elemente aufweist, die entlang der Länge darin oder darauf in einem sich wiederholenden Muster angeordnet sind, wobei der Magnetdetektor so angepasst ist, dass er Detektorsignale erzeugt, wenn sich das Kabel (204) während der Verwendung an dem Magnetdetektor vorbeibewegt.

**28.** System nach irgendeinem der Ansprüche 19 bis 22, wobei die Umwandlungseinheit ein oder mehr drehbare Teile, wie ein oder mehr Räder oder Kugeln, umfasst, die so angepasst sind, dass sie das Kabel (204) berühren und dadurch während der Verwendung gedreht werden, und eine elektromechanische Vorrichtung, angepasst um die Detektorsignale in Abhängigkeit von der Drehung der drehbaren Teile/des drehbaren Teils zu erzeugen.


**Revendications**

**1.** Système pour le traitement de structures anatomiques creuses, le système comprenant un applicateur (1) pour appliquer un rayonnement aux structures anatomiques creuses, l'applicateur (1) comprenant :

un élément allongé qui comprend un émetteur, l'émetteur étant couplé à une source de rayonnement hyperfréquence et étant conçu pour émettre ledit rayonnement, et l'émetteur comprenant :

une partie émettrice de rayonnement (208) composée d'un matériau diélectrique et ayant un axe d'allongement, la partie émettrice de rayonnement (208) étant façonnée et dimensionnée de sorte à émettre ledit rayonnement à une intensité prédéterminée dans un champ de dimensions limitées adjacent à celle-ci, moyennant quoi l'occlusion du tissu d'une structure anatomique creuse à l'intérieur dudit champ est accomplie de manière efficace, et

un conducteur allongé (210) à l'intérieur de la partie émettrice de rayonnement (208) et s'étendant au moins partiellement le long de celle-ci,

**caractérisé en ce que** le système comprend en outre :

un capteur de vitesse de mouvement (1104) conçu, en cours d'utilisation, pour détecter la vitesse de mouvement de l'applicateur (1) ; et

une unité de contrôle (14, 1102, 1344, 1444) couplée au capteur (1104) pour recevoir les signaux de vitesse de mouvement ainsi produits,

dans lequel l'unité de contrôle (14, 1102, 1344, 1444) est configurée pour calculer la vitesse de mouvement de l'applicateur (1) au moyen desdits signaux de vitesse de mouvement, et contrôler la quantité de rayonnement fournie à l'applicateur (1) et/ou la vitesse de mouvement de l'applicateur (1) en fonction de ladite vitesse de mouvement calculée.

2. Système selon la revendication 1, dans lequel la partie émettrice de rayonnement (208) comprend une partie effilée généralement conique (216), la partie effilée (216) formant ainsi une pointe (217) pour insertion dans une structure anatomique creuse.

3. Système selon la revendication 2, dans lequel le conducteur allongé (210) s'étend sur l'ensemble de la longueur de la partie émettrice de rayonnement (208), moyennant quoi ledit champ est disposé, en cours d'utilisation, sensiblement autour de ladite pointe (217).

4. Système selon la revendication 2, dans lequel le conducteur allongé (210) s'étend partiellement le long de la longueur de la partie émettrice de rayonnement (208), moyennant quoi, ledit champ est disposé, en cours d'utilisation, sensiblement autour de la section intermédiaire de ladite partie émettrice de rayonnement (208) et espacé par rapport à ladite pointe (217).

5. Système selon l'une quelconque des revendications précédentes, dans lequel un capteur de température (902) est prévu sur ledit élément allongé, ledit capteur de température (902) comprenant de préférence un thermocouple ou un capteur à fibre optique.

6. Système selon l'une quelconque des revendications 2 à 5, dans lequel la partie émettrice de rayonnement (208) comprend une partie sensiblement cylindrique (215) solidaire de la partie effilée (216).

7. Système selon l'une quelconque des revendications précédentes, dans lequel, pour calculer la vitesse de mouvement de l'applicateur (1), l'unité de contrôle (14, 1102, 1344, 1444) est configurée pour interroger le capteur de vitesse de mouvement (1104), l'intervalle d'interrogation entre les interrogations successives étant de durée uniforme ; déterminer une valeur de différence, la valeur de différence étant une différence entre les comptages définis par les signaux de vitesse de mouvement successifs ; utiliser la valeur de différence déterminée et un facteur de conversion R, calculer la vitesse de mouvement de l'applicateur (1) en utilisant R et la valeur de différence.

8. Système selon l'une quelconque des revendications précédentes, dans lequel la vitesse de mouvement est calculée en utilisant v = (cj-cj,) R où (ci-ci-1) est la valeur de différence.

9. Système selon l'une quelconque des revendications précédentes, dans lequel l'applicateur (1) est monté sur l'extrémité d'un câble allongé (204) et la vitesse de mouvement de l'applicateur (1) est calculée en calculant la vitesse de mouvement du câble (204).

10. Système selon la revendication 9, dans lequel l'intervalle d'interrogation entre les interrogations successives est T et le facteur de conversion est déterminé en tant que R = 1/KT, où K est une constante de conversion de comptage prédéterminée pour le câble (204).

11. Système selon la revendication 5 telle qu'annexée à la revendication 9 ou la revendication 10, dans lequel le câble (204) est un câble coaxial et une partie dudit câble (204) en butée avec la partie émettrice de rayonnement (208) est entourée, et fixée à celle-ci, par une ferrule conductrice (206) ; et dans lequel le capteur de température (902) est disposé sur la ferrule (206).

EP 1 675 519 B1

**12.** Système selon la revendication 11, dans lequel une série de marquages espacés de manière régulière (1218) sont prévus sur la surface extérieure du câble coaxial (204) le long de sa longueur.

**13.** Système selon la revendication 11, dans lequel les marquages (1218) sont espacés de manière irrégulière au lieu d'être espacés de manière régulière.

**14.** Système selon la revendication 12 ou la revendication 13, dans lequel les marquages (1218) comprennent en alternance des sections de couleurs claire (702) et foncée (704).

**15.** Système selon la revendication 14, dans lequel les sections de couleurs claire (702) et foncée (704) font chacune environ 1 cm de long.

**16.** Système selon l'une quelconque des revendications précédentes, dans lequel le système comprend en outre un dispositif d'affichage (20) conçu pour afficher, sous le contrôle de l'unité de contrôle (14, 1102, 1344, 1444), la vitesse de mouvement calculée de l'applicateur (1).

**17.** Système selon la revendication 16, dans lequel le dispositif d'affichage (20) est conçu pour afficher, sous le contrôle de l'unité de contrôle (14, 1102, 1344, 1444), une représentation graphique (1560) de la vitesse de mouvement calculée de l'applicateur (1).

**18.** Système selon la revendication 17, dans lequel ladite représentation graphique (1560) comprend une représentation graphique de type compteur de vitesse.

**19.** Système selon l'une quelconque des revendications 9 à 18, dans lequel le capteur de vitesse de mouvement (1104) comprend un logement (1206) par rapport auquel, en cours d'utilisation, le câble (204) se déplace, une unité de détection (1232) disposée à l'intérieur du logement (1206), l'unité de détection (1232) comprenant un dispositif de conversion conçu pour générer des signaux de détection provoqués par le mouvement de l'article, et des circuits de traitement (1234) conçus pour recevoir lesdits signaux de détection et produire des signaux de mouvement indicatifs de la vitesse de mouvement de l'article.

**20.** Système selon la revendication 19, dans lequel le logement (1206) comprend au moins une ouverture permettant le mouvement du câble (204) par rapport au logement (1206).

**21.** Système selon la revendication 20, dans lequel le logement (1206) a une configuration grâce à laquelle, en cours d'utilisation, le mouvement du câble (204) dans le logement (1206) ou près de celui-ci est sensiblement linéaire.

**22.** Système selon la revendication 20 ou 21, dans lequel ladite au moins une ouverture comprend une ouverture d'entrée à travers laquelle, en cours d'utilisation, le câble (204) pénètre dans le logement (1206) et une ouverture de sortie à travers laquelle, en cours d'utilisation, le câble (204) sort du logement (1206), le câble (204) se déplaçant de préférence, en cours d'utilisation, selon une trajectoire sensiblement linéaire entre lesdites ouverture d'entrée et ouverture de sortie.

**23.** Système selon l'une quelconque des revendications 19 à 22, dans lequel le dispositif de conversion comprend au moins un détecteur de rayonnement conçu pour recevoir le rayonnement du câble (204) et générer des signaux de détection en fonction dudit rayonnement reçu.

**24.** Système selon la revendication 23, dans lequel le rayonnement est un rayonnement optique, l'unité de détection (1232) comprend en outre un émetteur optique pour émettre le rayonnement optique et le détecteur de rayonnement est disposé de sorte à recevoir ledit rayonnement optique après réflexion depuis le câble (204).

**25.** Système selon la revendication 24, dans lequel l'émetteur optique est une LED, et de préférence dans lequel l'émetteur optique et le détecteur de rayonnement comprennent un dispositif intégré.

**26.** Système selon la revendication 23, dans lequel le détecteur de rayonnement comprend un détecteur de radioactivité de niveau faible, et le câble (204) a une pluralité d'éléments radioactifs disposés à l'intérieur ou sur celui-ci selon un motif répétitif le long de sa longueur.

**27.** Système selon l'une quelconque des revendications 19 à 22, dans lequel l'unité de conversion comprend un détecteur magnétique et le câble (204) a une pluralité d'éléments magnétiques disposés à l'intérieur ou sur celui-ci selon un motif répétitif le long de sa longueur, le détecteur magnétique étant conçu pour générer lesdits signaux de détection lorsque le câble (204), en cours d'utilisation, se déplace devant le détecteur magnétique.

28. Système selon l'une quelconque des revendications 19 à 22, dans lequel l'unité de conversion comprend un ou plusieurs élément(s) rotatif(s), tel(s) qu'une ou plusieurs roues ou billes, conçu(s) pour entrer en contact avec le câble (204) et être ainsi tourné(s), en cours d'utilisation, et un dispositif électromécanique conçu pour générer lesdits signaux de détection en fonction de la vitesse de rotation dudit(desdits) élément(s) de rotation.

Fig.1
(prior art)

Fig.2(a)

Fig.2(b)

Fig.2(c)

Fig.3

Fig.4(a)

Fig.4(b)

Fig.4(c)

Fig.5

Fig.6(a)

Fig.6(b)

204

## Fig.7(a)

204

702

604

## Fig.7(b)

A

706    708    204

## Fig.7(c)

204    1    802

B

**Fig.8(a)**

804

B

**Fig.8(b)**

1    208

C

804

**Fig.8(c)**

204

C

**Fig.8(d)**

1    1,5

214    906

908    206    902 904

⊞ Dielectric
▨ FEP
▦ Coaxial cable
☐ Aluminium ferrute

**Fig.9**

Fig.11

Fig.10(a)

Monitor Treatment

Fig.10(b)

**Fig.12(a)**

**Fig.12(b)**

**Fig.12(c)**

1340

1346

Interface

1344 ⇕ I/O

1342

Power
Supply Unit

Power →

Control
Module

Serial Data

1104

Applicator
Motion
Sensor

1338

Surface Image

1348 ⇕ I/O

Applicator
Power
Module

Ref Power

Applicator

Fwd Power →

1

1350 ↑ Enable

Footswitch

# Fig.13

Master Device

System Control Unit

1452

Application
Software

1438

RS232 Serial Link

1444

Slave Device

Applicator Motion Sensor

1434

Microchip
Micro-
controller

1432

Optical
Motion
Sensor

1104

# Fig.14

1574          1576

Total distance 37-8mm    Time elapsed: 140s

End    STATUS:         POWER  ON

1560    1564    15.0    17.5←1562    20.0    cm/min

12.5    22.5    1568

1566    25.0

10.0

1570    1572

7.5    27.5

## Fig.15

-204b

-1104'

204b    Sensor
housing

Paralell cable tethered to
applicator cable

Into patient

204a    204a

──────── Applicator cable
⌒⌒⌒⌒⌒⌒ Cable in parallel

## Fig.16

Fig.17(a)

Fig.17(b)

| $P_1$ | $P_2$ | $P_3$ | $P_4$ | $P_5$ | $P_6$ | $P_7$ | ... | $P_N$ |
|---|---|---|---|---|---|---|---|---|
| $C_1$ | $C_2$ | $C_3$ | $C_4$ | $C_5$ | $C_6$ | $C_7$ | ... | $C_N$ |
| | $(C_2-C_1)R$ | $(C_3-C_2)R$ | $(C_4-C_3)R$ | $(C_5-C_4)R$ | $(C_6-C_5)R$ | $(C_7-C_6)R$ | ... | $(C_N-C_{N-1})R$ |

Fig.17(c)

Fig.18

EP 1 675 519 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1103228 A
- WO 9956642 A
- WO 0049957 A
- WO 9956643 A
- US 5800494 A
- US 5810803 A
- US 5693082 A
- WO 9504385 A